# EUROPEAN PATENT SPECIFICATION

(11) **EP 2 195 448 B1**
(45) Date of publication and mention of the grant of the patent: **13.03.2013**
(21) Application number: 07808542.0
(22) Date of filing: 20.08.2007
(51) Int. Cl.: C12Q 1/68

(54) **METHOD TO PREDICT IRIS COLOR**
VERFAHREN ZUR VORHERSAGE DER IRISFARBE
PROCÉDÉ DE PRÉDICTION DE LA COULEUR DE L'IRIS

(43) Date of publication of application: 16.06.2010
(73) Proprietor: Erasmus University Medical Center Rotterdam, 3015 GE Rotterdam (NL)
(72) Inventor: KAYSER, Manfred, Heinz, NL-3015 GE Rotterdam (NL); VAN DUIJN, Cornelia, Marja, NL-3015 GE Rotterdam (NL); OOSTRA, Bernard, Anne, NL-3015 GE Rotterdam (NL)
(74) Representative: Hatzmann, Martin
(86) International application number: PCT/NL2007/050409
(87) International publication number: WO 2009/025544

(56) References cited:
- WO-A-2005/079331
- US-A1- 2003 211 486
- DUFFY DAVID L ET AL: "A three-single-nucleotide polymorphism haplotype in intron 1 of OCA2 explains most human eye-color variation." AMERICAN JOURNAL OF HUMAN GENETICS FEB 2007, vol. 80, no. 2, February 2007 (2007-02), pages 241-252, XP002481702 ISSN: 0002-9297 cited in the application
- DATABASE SNPDB NCBI; 6 September 2000 (2000-09-06), XP002481703 Database accession no. rs916977
- DATABASE SNPDB NCBI; 30 June 2003 (2003-06-30), XP002481704 Database accession no. rs6497287
- DATABASE SNPDB NCBI; 4 July 2003 (2003-07-04), XP002481705 Database accession no. rs8028689
- DATABASE SNPDB NCBI; 4 July 2003 (2003-07-04), XP002481706 Database accession no. rs8041209
- FRUDAKIS TONY ET AL: "Multilocus OCA2 genotypes specify human iris colors." HUMAN GENETICS NOV 2007, vol. 122, no. 3-4, 7 July 2007 (2007-07-07), pages 311-326, XP019563956 ISSN: 0340-6717 cited in the application
- FRUDAKIS TONY ET AL: "Sequences associated with human iris pigmentation." GENETICS DEC 2003, vol. 165, no. 4, December 2003 (2003-12), pages 2071-2083, XP008040049 ISSN: 0016-6731 cited in the application

## Description

This invention relates to the field on human genetics, more particularly to the prediction of iris color on basis of polymorphism markers.

Human iris color (also referred to as eye color) was one of the first human traits used to investigate Mendelian inheritance (Davenport and Davenport, 1907) but is now considered a polygenetic trait (Frudakis et al., 2003; Sturm and Frudakis, 2004). The physical basis of iris color variation is the amount of melanin pigment and the number of melanosomes in the outermost layer of the iris (anterior iridal stroma). Brown irides have more melanin pigment and more melanosomes than blue ones, whereas the number of melanocytes is similar in irides of both color types (Imesch et al., 1996; Sturm and Frudakis, 2004; Wielgus and Sarna, 2005). The melanin pigment in the melanosomes can occur in two forms: eumelanin, a brown-black form responsible for dark iris colors, and pheomelanin, a red-yellow form of melanin (Sturm and Frudakis, 2004). Human iris color remains fairly constant past early childhood, but can be subject to adrenergic regulation past childhood and can also change due to medication (Bito et al., 1997; Imesch et al., 1997). Most human populations around the world have brown iris color while blue and green colors are additionally found in people of European descent. This makes Europeans the target population for investigating the genetic basis of iris color variation.

Early genetic studies of human iris color variation have targeted two genomic regions, one on chromosome 19 (Eiberg and Mohr, 1987) and the other on chromosome 15 (Eiberg and Mohr, 1996). The chromosome 15 region was also confirmed in two recent, independent, microsatellite-based genome scans in twins (Zhu et al., 2004; Posthuma et al., 2006). This region harbors the OCA2 gene, which represents the human homologue of the mouse pink eye dilution locus, encodes the P-protein and is involved in Type II oculocutaneous albinism (Ramsay et al., 1992; Rinchik et al., 1993; Spritz et al., 1995; Brilliant, 2001). Several Single Nucleotide Polymorphisms (SNPs) within *OCA2* were associated with iris color in humans including two coding variants (Rebbeck et al., 2002; Frudakis et al., 2003; Duffy et al., 2004). Recently, the *OCA2* gene was characterized in more detail. Duffy et al. (Duffy et al,. 2007) studied 71 *OCA2* SNPs, most of them tagging SNPs derived from the HapMap project, in about 3,800 twins, their siblings and parents. The strongest association was observed for three SNPs from intron 1 with P values of 10-54 to 10-96 whereas other *OCA2* SNPs showed less association (<10-20). However, the high association signal in intron 1 appeared to extend into the 5' proximal region outside the *OCA2* gene suggesting unknown variants located outside the *OCA2* gene determining iris color variation in humans which was not investigated by Duffy et al. (2007). Frudakis et al. (Frudakis et al., 2007) investigated a total of 395 *OCA2* SNPs in two partly overlapping sets of individuals and reported 33 SNPs in association with iris color of which six were identified previously (Frudakis et al., 2003) and two match the intron 1 SNPs identified by Duffy et al. (Duffy et al., 2007). Other genes that have been implicated in iris color include *MATP, ASIP, TYRP1, CYP1A2, CYP2C8, and CYP2C9* (WO 2002/097047; Kanetsky et al., 2002; Frudakis et al., 2003; Graf et al., 2005).

There is, however, still need for better markers for iris color. Such markers can advantageously be used in forensic investigations and other application of human identification where nucleic acid samples e.g. from a crime scene are used to obtain information on the subjects who have left behind this nucleic acid at the crime spot. Currently, human identification using nucleic acid markers is completely comparative and compares marker profiles (DNA fingerprints, DNA profiles) obtained from crime scene samples with those obtained from known suspects. If no suspect is known to the police no profile can be obtained and compared with the one collected from the crime scene. Consequently, in such cases the person who left the sample at the crime scene and who might have committed the crime can not be identified using genetic (DNA) evidence. Similarly, missing persons are currently identified be comparing a DNA profile obtained from their remains with that obtained from a known relative. If nothing is known about the missing person, no relatives can be identified for genetic testing and no DNA profile is available for comparison. If one would have nucleic acid markers that could reliably predict eye (iris) color this would help in finding unknown persons (suspects / missing persons) in a direct way and without comparing DNA profiles.

### SUMMARY OF THE INVENTION

The inventors now have found several markers in a hitherto uninvestigated area, the genomic region 5' proximal (outside) of the *OCA2* gene and including the direct neighbouring *HERC2 gene.* This region is located on chromosome 15 between basepairs 26018062 and 26240890 (according to NCBI build 36 or Ensemble Homo sapiens version 46.36h). Accordingly, the invention relates to a method to predict eye color of a human e.g. an unknown person from a nucleic acid / protein sample by assaying for one or more polymorphisms in the *HERC2* gene wherein the one or more polymorphisms are selected from the group consisting of rs916977, rs8028689, rs6497287, rs8041209, rs6497292, rs2240202, rs2346050, rs12592730, rs7183877, rs2240204, rs8039195, rs16950979, rs16950987, rs1667394, and rs1635168 and any marker in the HERC2 gene in genetic linkage disequilibrium with said polymorphisms.

Preferably the polymorphism is rs916977, where the prediction is blue eyes when the polymorphism has the nucleotide sequence CC (or GG considering the complementary bases), or brown eyes when the nucleotide sequence is TT (or AA considering the complementary bases). For the other markers and their alleles the prediction goes as follows with both alleles from both homologue chromosomes provided and the complementary bases indicated in brakeage: rs8028689: CC (GG) brown and TT (AA) blue, rs6497287: AA (TT) blue and GG (CC) brown, rs8041209: AA (TT) brown and CC (GG) blue, rs6497292: AA (TT) blue and GG (CC) brown, rs2240202: AA (TT) brown and GG (CC) blue, rs2346050: AA (TT) blue and GG (CC) brown, rs12592730: AA (TT) brown and GG (CC) blue, rs7183877: TT (AA) blue and GG (CC) brown, rs2240204: TT (AA) brown and CC (GG) blue, rs8039195: TT (AA) blue and CC (GG) brown, rs16950979: AA (TT) blue and CC (GG) brown, rs16950987: TT (AA) brown and CC (GG) blue, rs1667394: TT (AA) blue and CC (GG) brown, and rs1635168: TT (AA) brown and GG (CC) blue.

### LEGENDS TO THE FIGURES

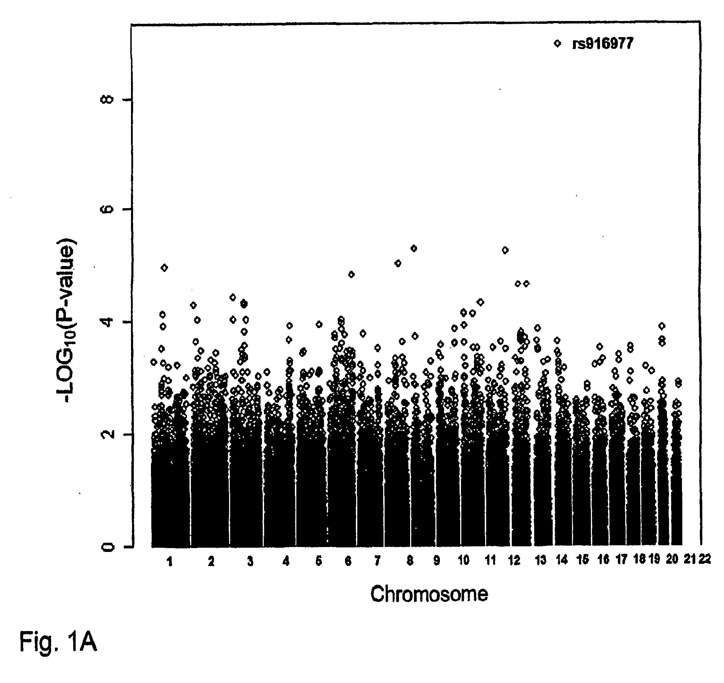

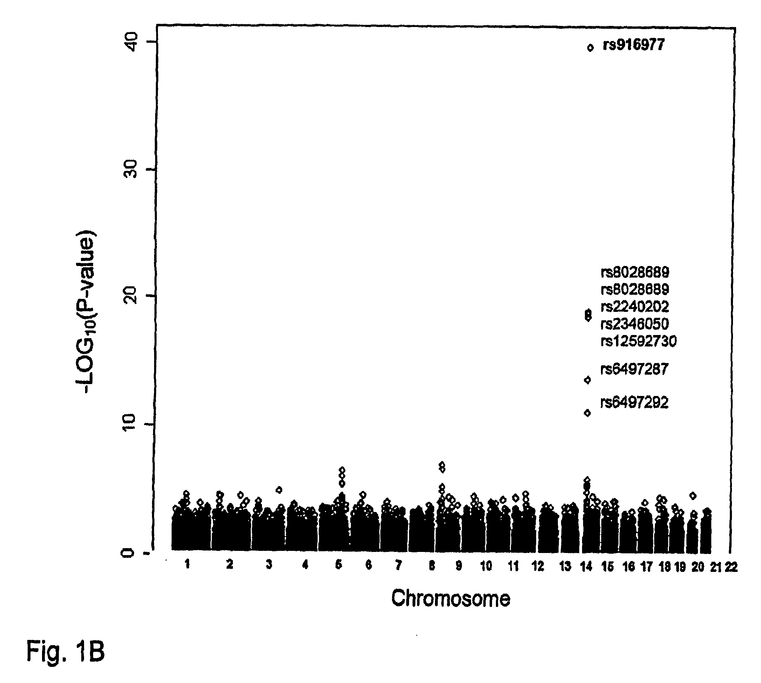

**Figure 1****.** Genome-wide association for human iris color in two independent population samples from The Netherlands: A) the ERF (Erasmus Rucphen Family) study (N=192) using Affymetrix 250K SNPs, and B) the Rotterdam study (N=481) using Affymetric 500K SNPs taking into account multiple testing. SNPs associated with iris color at the genome-wide statistical significance level of <0.001 are named, corresponding markers between both studies are highlighted in bold.

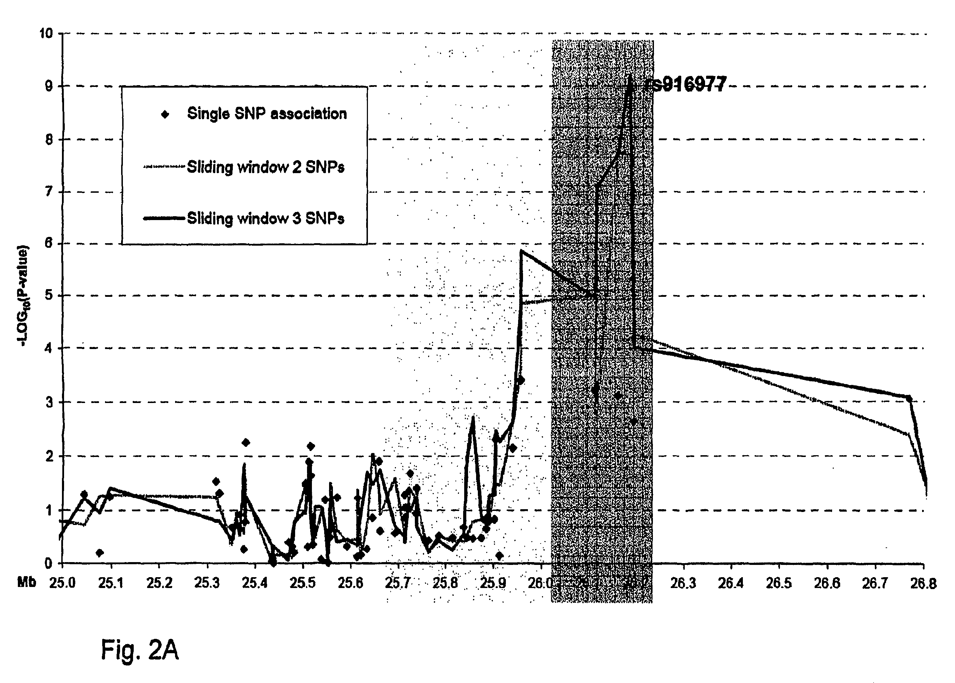

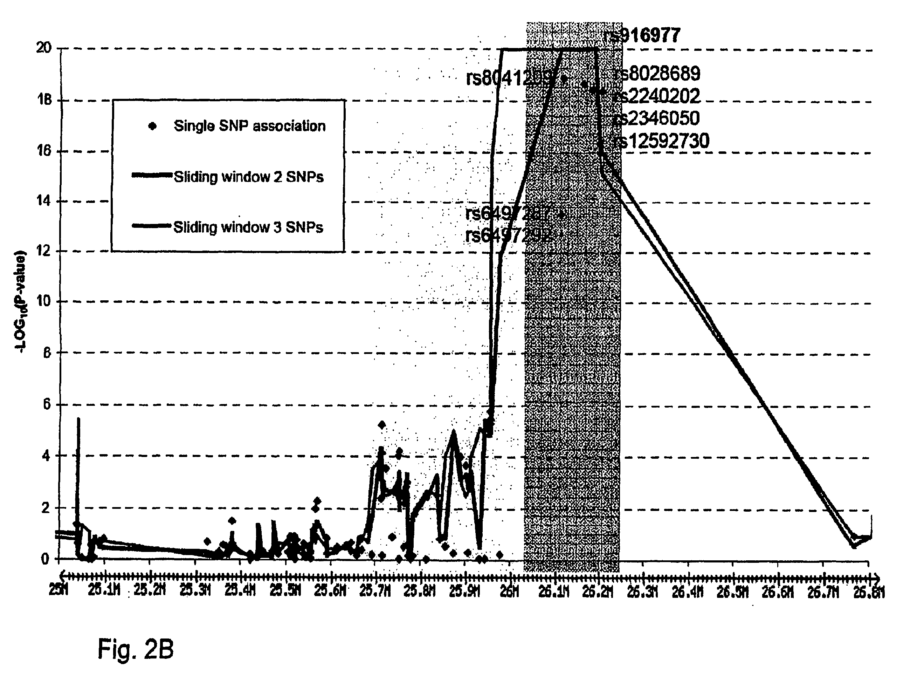

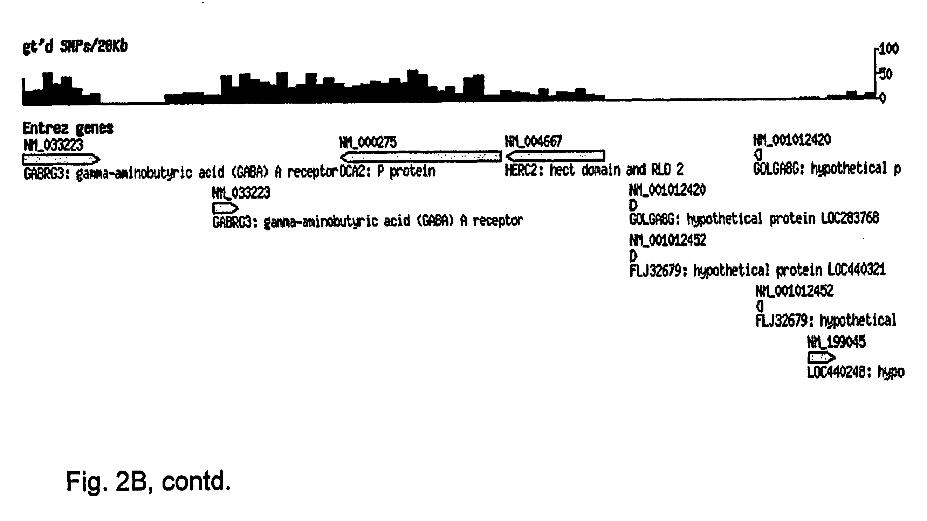

**Figure 2****.** SNP association with iris color from region 25-26.8 Mb of chromosome 15 from two independent GWA studies from The Netherlands: A) the ERF study (N=192), and B) the Rotterdam study (N=481). Chi-square test statistics (Y-axis) are plotted against the physical map distance, aligned with known SNPs and genes in the region. The *OCA2* and the *HERC2* genes are highlighted with green and blue boxes respectively. The score of the -log10 P-values for each SNP association with blue/nonblue eye color is shown as unconnected blue dots, and haplotype associations based on sliding window of two and three SNPs are depicted as continuous pink and orange lines respectively. The -log 10 P-values are truncated at 20. SNPs that reached the genome-wide significance level of 0.05 are named.
   Corresponding markers between both studies are highlighted in bold.

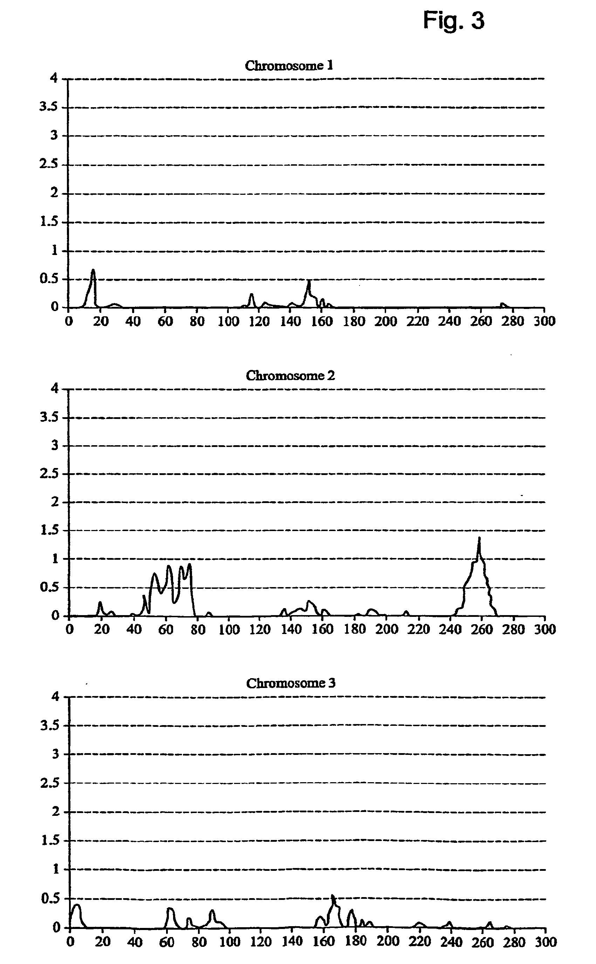

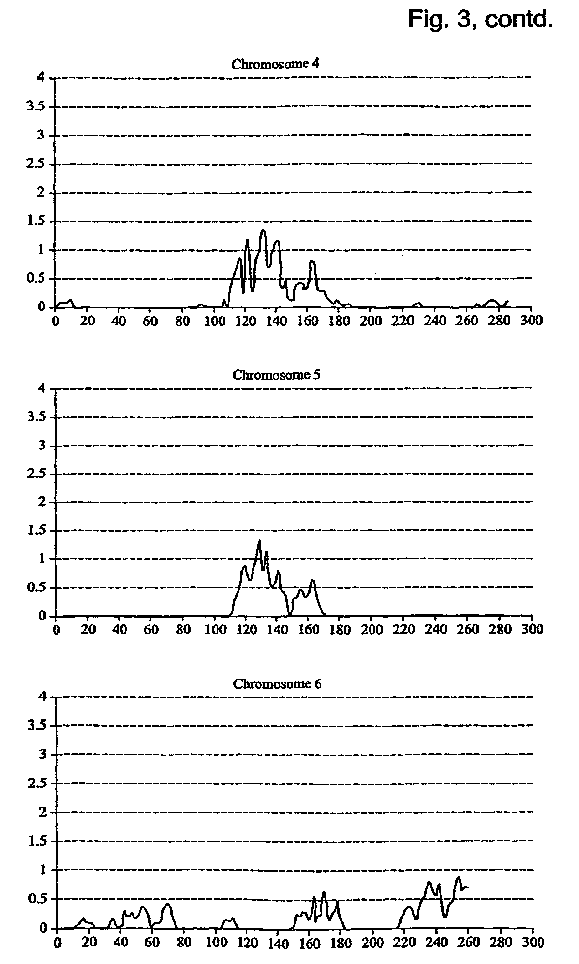

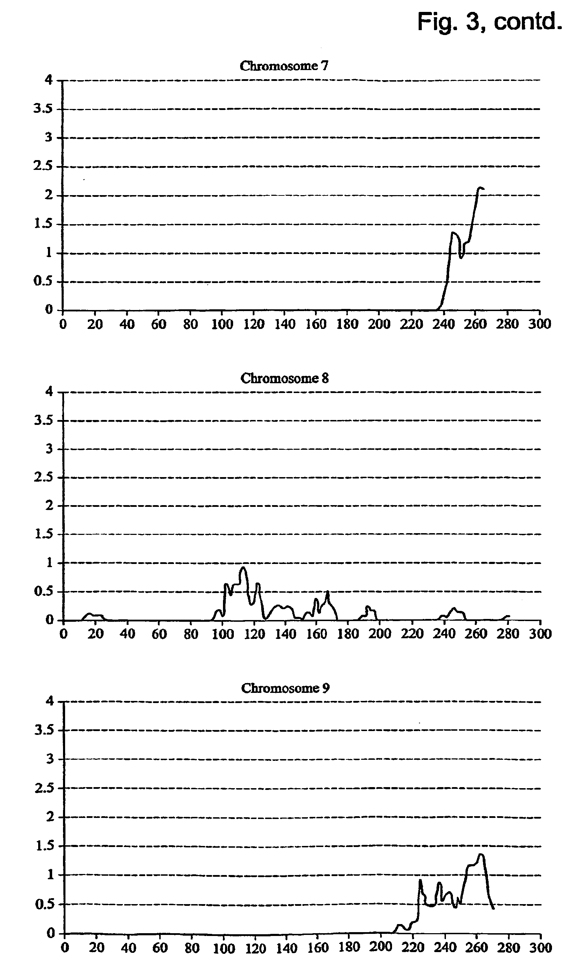

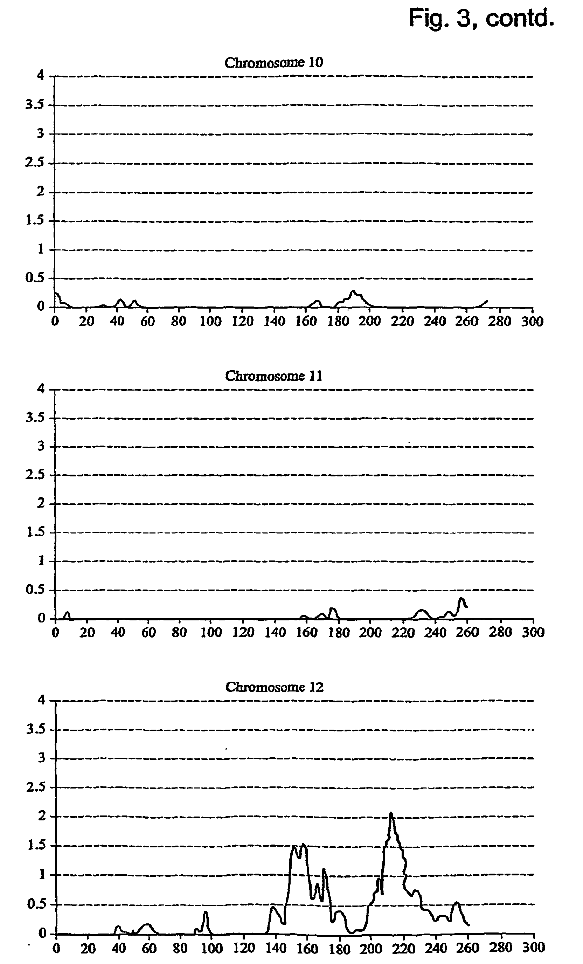

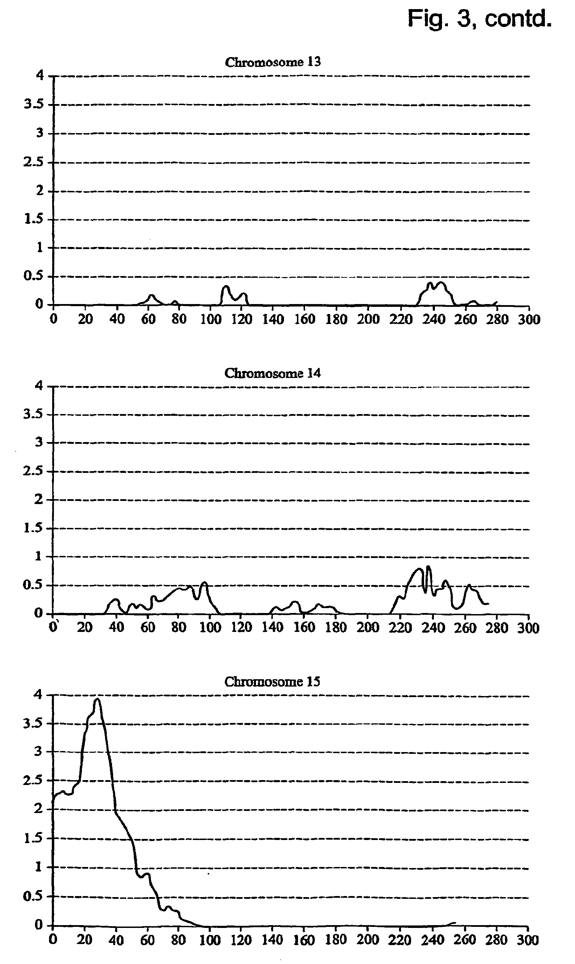

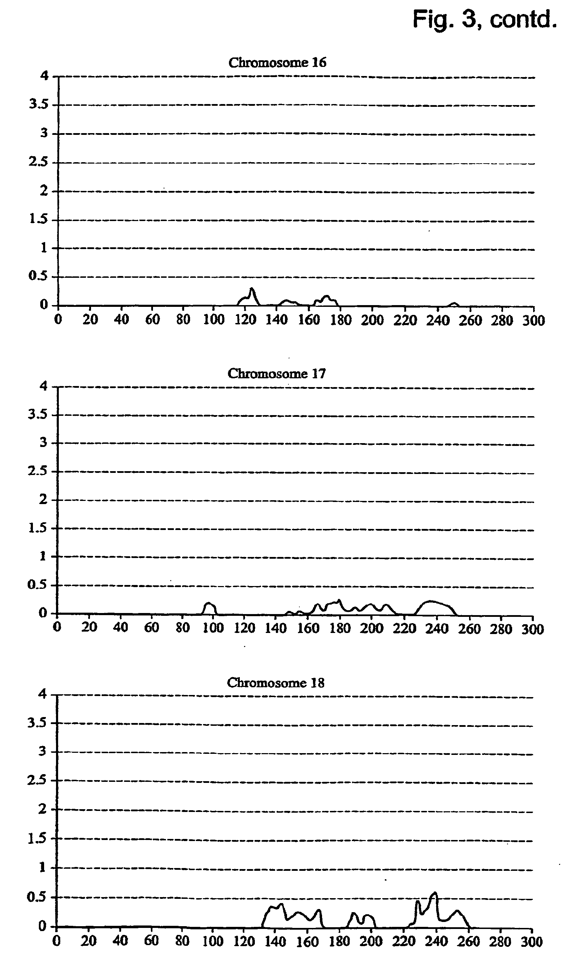

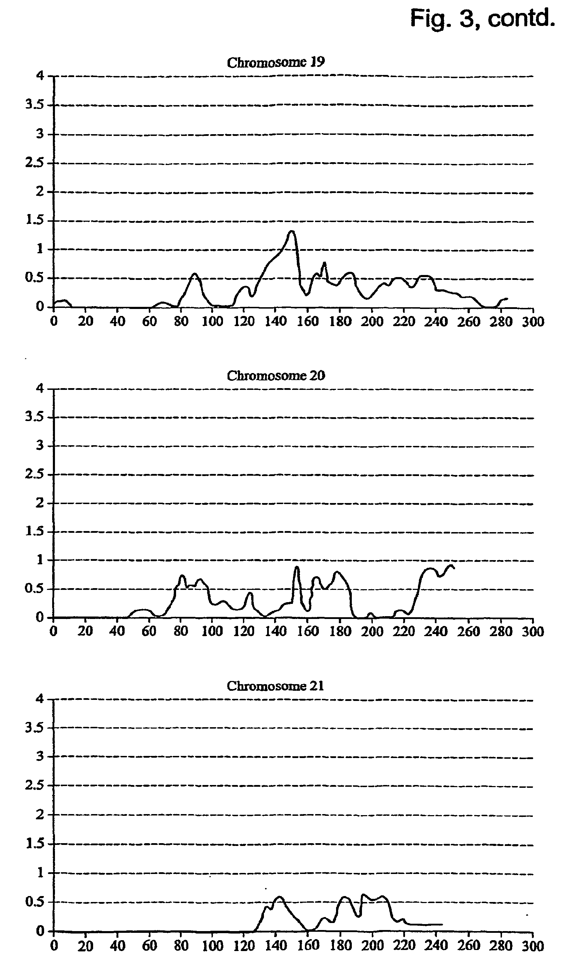

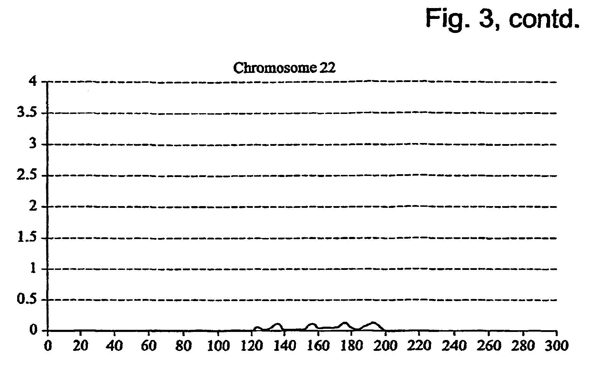

Figure 3. Results from genome-wide linkage analyses for human iris color. LOD scores are shown per chromosome.

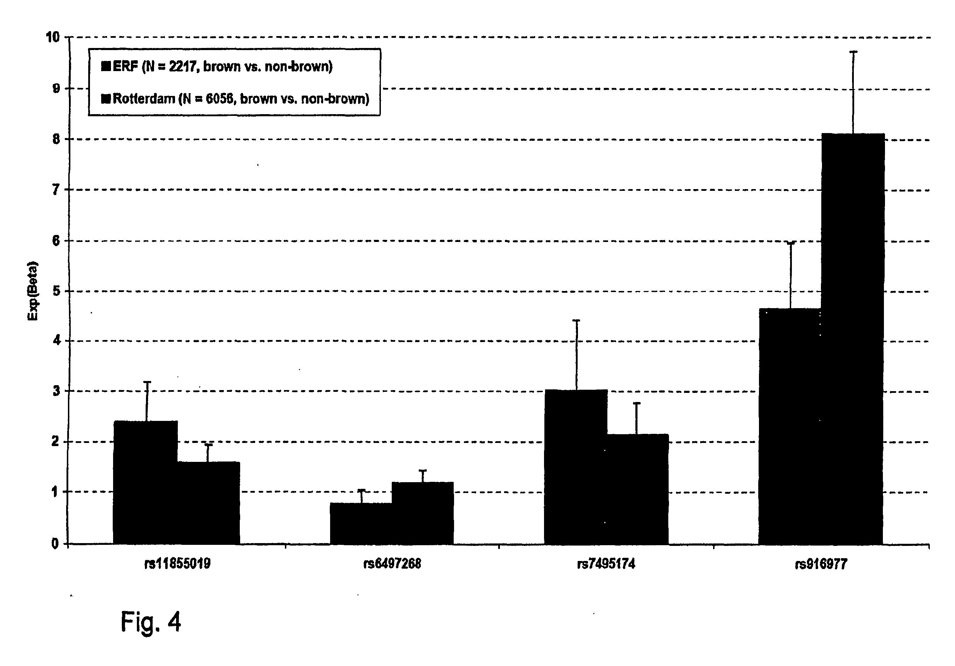

Figure 4. ODDs ratios (OR) for brown iris color for four SNPs: rs11855019, rs6497268, rs7495174 in the *OCA2* and rs916977 in the *HERC2* gene in the ERF (N=2217) and the Rotterdam study populations (N=6056). OR were derived using logistic regression (binary brown and non-brown iris color against the number of minor alleles of each SNP) where all four SNPs were adjusted in the same model.

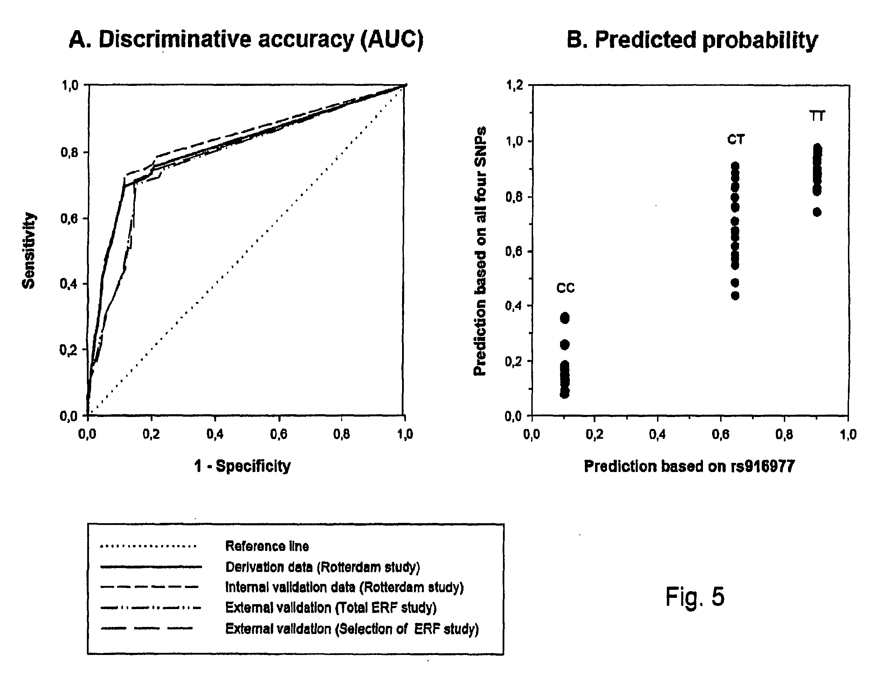

**Figure 5****.** Discriminative accuracy and prediction probabilities of brown iris color using genotypes of four SNPs: rs11855019, rs6497268, rs7495174 in the *OCA2* and rs916977 in the *HERC2* gene in the ERF (N=2217) and the Rotterdam study population (N=6056): A)
   discriminative accuracy for the prediction of brown iris color based on all four SNPs in different sample sets, B) comparison of prediction probabilities for brown iris color from all four SNPs with that based on rs916977 alone.

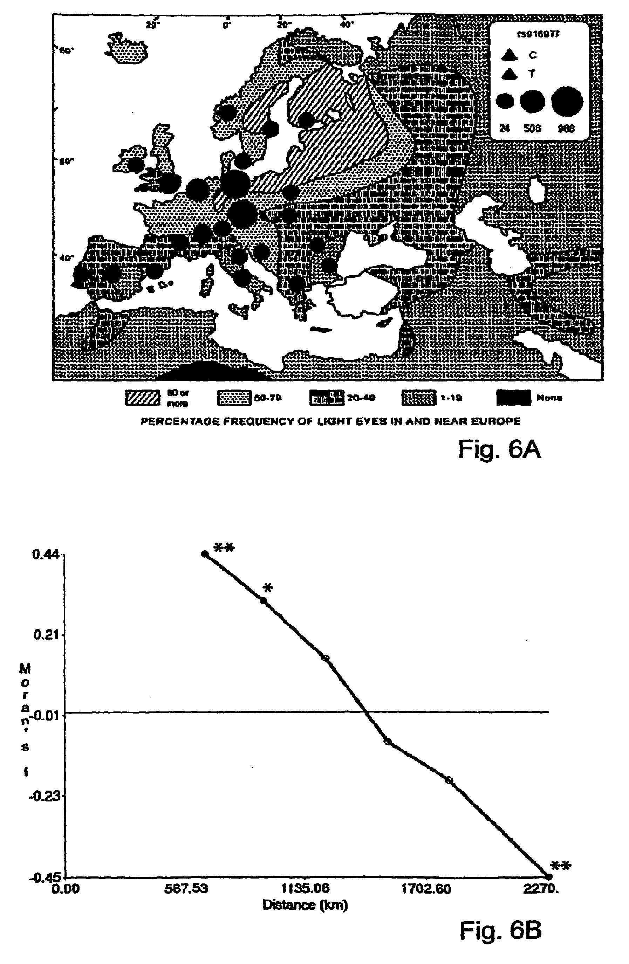

**Figure 6****.** Allele frequency distribution of rs916977 across Europe: A) rs916977 allele frequencies for 23 European populations (including the Rotterdam study) superimposed on a map of Europe indicating classes of human iris color. Sizes of the pie charts indicate sample size. B) Spatial autocorrelation analysis of rs916977 in Europe considering 23 population samples. The shape of the autocorrelogram describes the geographical pattern of rs916977 allelic frequencies. Positive Moran's I values for short distances and negative for large geographic distances indicate a clinal pattern of the genetic data, starting from one side of the map to the opposite one. Figure 6A (without data on rs916977) is from Beals and Hoijer "An Introduction To Anthropology", 3rd edition, published by Allyn and Bacon, Boston, MA. Copyright © 1965 by Pearson Education.

### DETAILED DESCRIPTION OF THE INVENTION

To date, all genome wide searches for genes involved in human iris color have been based on linkage studies, targeting relatively rare genes with large effects and revealing large genomic regions, and candidate gene studies, targeting genes which, based on their function, are expected to be involved in iris color pigmentation. Genome-wide association (GWA) has been proven to be a powerful approach to study common variants involved in traits. So far, no GWA has been conducted for iris color in humans. Here, we applied both a GWA and linkage approach to human iris color variation in two independent studies. One GWA was conducted in a recent genetically isolated human population from the Netherlands, the Erasmus Rucphen Family (ERF) study, and the other in an outbred population sample from the Netherlands, the Rotterdam study.

The aim of our study was to unravel the major genes contributing to human iris color variation in Europeans together with evaluating their value of predicting iris color phenotypes of unknown persons, as this may become relevant in future forensic and other applications of human identification. Current ways of DNA-based human identification follow a strictly comparative approach of matching (or not) non-coding DNA profiles obtained from the crime scene (or a missing person) with that of a known suspect (or known relatives of the missing person). If suspects (or relatives) are unknown a forensic case can not be solved with the help of currently used DNA evidence. Predicting visible traits such as iris color of an unknown person using informative genetic markers, if done reliably, would help in tracing unknown persons.

It appeared from our studies that the region 5' proximal of the *OCA2* gene up to and including the *HERC2* gene is the most important region involved in iris color from two independent population genetics studies. This region is located on chromosome 15 between basepairs 26018062 and 26240890 according to NCBI build 36 or Ensemble Homo sapiens version 46.36h. Several SNPs were found in this region that discriminate for iris color between blue eyes, brown eyes and eyes of intermediate color. Of these SNPs, rs916977 in intron 11 of the *HERC2* gene is the SNP strongest associated with human iris color variation. This is the only marker reaching genome-wide significance in both GWA studies; there were no other regions showing consistent genome-wide significance in both GWA studies; there were no other regions showing consistent genome-wide evidence for iris color association. The *HERC2* intron 11 marker is located 169 kb 5' proximal of the *OCA2* gene, which was previously reported to be associated with human iris color (Rebbeck et al., 2002; Duffy et al., 2004; Jannot et al., 2005; Duffy et al., 2007; Frudakis et al., 2007). When analyzing the *HERC2* and the three most important *OCA2* SNPs reported in a previous study (rs11855019, rs6497268, and rs7495174), rs916977 of the *HERC2* gene showed the lowest P-value in both enlarged populations. Furthermore, of these three *OCA2* SNPs only two (rs11855019 and rs7495174) remained significantly associated to iris color when the *HERC2* rs916977 was included in the model. In our prediction models *HERC2* rs916977 was the one of two most important SNPs for predicting iris color. The SNPs in genes suggested earlier with involvement in iris color did not contribute *(MATP, ASIP, CYP1A2, CYP2C8, and CYP2C9)* or contributed marginally *(TYRP1).* Also the *OCA2* SNPs identified in the GWA of the Rotterdam study contributed only marginally to the prediction of iris color, when their predictive value was evaluated together with the other markers. Overall, genetic association with human iris color was higher for markers located in the *HERC2* gene that it was for markers from any other gene, including *OCA2.* It appeared that blue iris color was associated with the presence of the SNPs CC (complementary bases GG) in rs916977, while brown iris color was associated with the presence of the nucleotides TT (complementary bases AA). In addition, we found seven SNPs from the HERC2 gene with significant association with iris color on the genome-wide level in the Rotterdam study population with the following genotype - iris color association (considering both homologue chromosomes and the complementary bases in brakeage): rs8028689: CC (GG) brown and TT (AA) blue, rs6497287: AA (TT) blue and GG (CC) brown, rs8041209: AA (TT) brown and CC (GG) blue, rs6497292: AA (TT) blue and GG (CC) brown, rs2240202: AA (TT) brown and GG (CC) blue, rs2346050: AA (TT) blue and GG (CC) brown, rs12592730: AA (TT) brown and GG (CC) blue. Furthermore, we found seven other SNPs from the *HERC2* gene when applying the Illumina 300 Duo SNP arrays to 733 ERF participants and one (rs8028689) overlapping with the results from Affymetrix 500K array in the Rotterdam study with the following genotype-iris color association (considering both homologue chromosomes and the complementary bases in brakeage): rs7183877: TT (AA) blue and GG (CC) brown, rs2240204: TT (AA) brown and CC (GG) blue, rs8039195: TT (AA) blue and CC (GG) brown, rs16950979: AA (TT) blue and CC (GG) brown, rs16950987: TT (AA) brown and CC (GG) blue, rs1667394: TT (AA) blue and CC (GG) brown, and rs1635168: TT (AA) brown and GG (CC) blue.

"Polymorphisms" are allelic variants that occur in a population. The polymorphism can be a single nucleotide difference present at a locus, or can be an insertion or deletion of one or a few nucleotides. As such, a single nucleotide polymorphism ("SNP") is characterized by the presence in a population of one or two, of four nucleotides (i. e., adenosine, cytosine, guanosine or thymidine) at a particular locus in a genome such as the human genome. Accordingly, it will be recognized that, while the methods of the invention are exemplified primarily by the detection of SNPs, the disclosed methods or others known in the art similarly can be used to identify other polymorphisms in the exemplified genetic region of the HERC2 gene in genetic linkage desequilibrium with said SNPs.

Haplotypes can be inferred from genotype data corresponding to certain SNPs using the Stephens and Donnelly algorithm (Am. J. Hum. Genet. 68: 978-989,2001). Haplotype phases (i. e., the particular haplotype alleles in an individual) can also be determined using the Stephens and Donnelly algorithm. Software programs are available which perform this algorithm (e. g., The PHASE program, Department of Statistics, University of Oxford).
In one example, called the Haploscope method (See WO 02/0483 18 a candidate SNP combination is selected from a plurality of candidate SNP combinations for a gene associated with a genetic trait. Haplotype data associated with this candidate SNP combination are read for a plurality of individuals and grouped into a positive-responding group and a negative-responding group based on whether predetermined trait criteria for an individual are met. A statistical analysis (as discussed below) on the grouped haplotype data is performed to obtain a statistical measurement associated with the candidate SNP combination. The acts of selecting, reading, grouping, and performing are repeated as necessary to identify the candidate SNP combination having the optimal statistical measurement. In one approach, all possible SNP combinations are selected and statistically analyzed. In another approach, a directed search based on results of previous statistical analysis of SNP combinations is performed until the optimal statistical measurement is obtained. In addition, the number of SNP combinations selected and analyzed may be reduced based on a simultaneous testing procedure.

As used herein, the term "infer" or "inferring", when used in reference to a prediction of iris color, means drawing a conclusion about the eye color of a subject using a process of analyzing individually or in combination nucleotide occurrence(s) of one or more SNP(s) of the invention in a nucleic acid sample of the subject, and comparing the individual or combination of nucleotide occurrence(s) of the SNP(s) to known relationships of nucleotide occurrence(s) of the iris color. As disclosed herein, the nucleotide occurrence(s) can be identified directly by examining nucleic acid molecules, or indirectly by examining a polypeptide encoded by a particular gene, for example, the *HERC2* gene, when the polymorphism is associated with an amino acid change in the encoded polypeptide or has regulatory effects.

Methods of performing such a comparison and reaching a conclusion based on that comparison are exemplified herein. The inference typically involves using a complex model that involves using known relationships of known alleles or nucleotide occurrences as classifiers. The comparison can be performed by applying the data regarding the subject's haplotype allele(s) to a complex model that makes a blind, quadratic discriminate classification using a variance-covariance matrix. Such a model is illustrated in the Examples.

To determine whether haplotypes are useful in an inference of eye color, numerous statistical analyses can be performed. Allele frequencies can be calculated for haplotypes and pair-wise haplotype frequencies estimated using an EM algorithm (Excoffier and Slatkin, Mol Biol Evol. 1995 Sep; 12 (5): 921-7). Linkage disequilibrium coefficients can then be calculated. In addition to various parameters such as linkage disequilibrium coefficients, allele and haplotype frequencies (within ethnic, control and case groups), chi-square statistics and other population genetic parameters such as Panmitic indices can be calculated to control for ethnic, ancestral or other systematic variation between the case and control groups. Markers/haplotypes with value for distinguishing the case matrix from the control, if any, can be presented in mathematical form describing any relationship and accompanied by association (test and effect) statistics. A statistical analysis result which shows an association of a SNP marker or a haplotype with eye color with at least a probability of insignificance less than 0.05 (or 5%), can be used to identify most informative markers / haplotypes. These statistical tools may test for significance related to a null hypothesis that a SNP allele or haplotype allele is not different between the groups. If the significance of this difference is low, it suggests the allele is not related to eye color. If the significance is high (p-value at least <0.05, preferable <0.01) it suggests the allele / haplotype is related to eye color.

The term "primer" as used herein refers to an oligonucleotide which is capable of annealing to the amplification target allowing a DNA polymerase to attach thereby serving as a point of initiation of DNA synthesis when placed under conditions in which synthesis of primer extension product which is complementary to a nucleic acid strand is induced, i.e., in the presence of nucleotides and an agent for polymerization such as DNA polymerase and at a suitable temperature and pH. The (amplification) primer is preferably single stranded for maximum efficiency in amplification. Preferably, the primer is an oligodeoxyribonucleotide. The primer must be sufficiently long to prime the synthesis of extension products in the presence of the agent for polymerization. The exact lengths of the primers will depend on many factors, including temperature and source of primer. A "pair of bi-directional primers" as used herein refers to one forward and one reverse primer as commonly used in the art of DNA amplification such as in PCR amplification.

The term "probe" refers to a single-stranded oligonucleotide sequence that will recognize and form a hydrogen-bonded duplex with a complementary sequence in a target nucleic acid sequence analyte or its cDNA derivative.

The terms "stringency" or "stringent hybridization conditions" refer to hybridization conditions that affect the stability of hybrids, e.g., temperature, salt concentration, pH, formamide concentration and the like. These conditions are empirically optimised to maximize specific binding and minimize nonspecific binding of primer or probe to its target nucleic acid sequence. The terms as used include reference to conditions under which a probe or primer will hybridise to its target sequence, to a detectably greater degree than other sequences (e.g. at least 2-fold over background). Stringent conditions are sequence dependent and will be different in different circumstances. Longer sequences hybridise specifically at higher temperatures. Generally, stringent conditions are selected to be about 5°C lower than the thermal melting point (Tm) for the specific sequence at a defined ionic strength and pH. The Tm is the temperature (under defined ionic strength and pH) at which 50% of a complementary target sequence hybridises to a perfectly matched probe or primer. Typically, stringent conditions will be those in which the salt concentration is less than about 1.0 M Na+ ion, typically about 0.01 to 1.0 M Na+ ion concentration (or other salts) at pH 7.0 to 8.3 and the temperature is at least about 30°C for short probes or primers (e.g. 10 to 50 nucleotides) and at least about 60°C for long probes or primers (e.g. greater than 50 nucleotides). Stringent conditions may also be achieved with the addition of destabilizing agents such as formamide. Exemplary low stringent conditions or "conditions of reduced stringency" include hybridization with a buffer solution of 30% formamide, 1 M NaCl, 1% SDS at 37°C and a wash in 2x SSC at 40°C. Exemplary high stringency conditions include hybridization in 50% formamide, 1 M NaCl, 1% SDS at 37°C, and a wash in 0.1x SSC at 60°C. Hybridization procedures are well known in the art and are described in e.g. Ausubel, F.M., Brent, R., Kingston, R.E., Moore, D.D.,Seidman, J.G., Smith, J.A., Struhl, K. eds. (1998) Current protocols in molecular biology. V.B. Chanda, series ed. New York: John Wiley & Sons.

Methods for detecting a nucleotide change can utilize one or more oligonucleotide probes or primers, including, for example, an amplification primer pair, that selectively hybridize to a target polynucleotide, which contains one or more SNP positions. Oligonucleotide probes useful in practicing a method of the invention can include, for example, an oligonucleotide that is complementary to and spans a portion of the target polynucleotide, including the position of the SNP, wherein the presence of a specific nucleotide at the position (i. e., the SNP) is detected by the presence or absence of selective hybridization of the probe. Such a method can further include contacting the target polynucleotide and hybridized oligonucleotide with an endonuclease, and detecting the presence or absence of a cleavage product of the probe, depending on whether the nucleotide occurrence at the SNP site is complementary to the corresponding nucleotide of the probe. A pair of probes that specifically hybridize upstream and adjacent and downstream and adjacent to the site of the SNP, wherein one of the probes includes a nucleotide complementary to a nucleotide occurrence of the SNP, also can be used in an oligonucleotide ligation assay, wherein the presence or absence of a ligation product is indicative of the nucleotide occurrence at the SNP site. An oligonucleotide also can be useful as a primer, for example, for a primer extension reaction, wherein the product (or absence of a product) of the extension reaction is indicative of the nucleotide occurrence. In addition, a primer pair useful for amplifying a portion of the target polynucleotide including the SNP site can be useful, wherein the amplification product is examined to determine the nucleotide occurrence at the SNP site.

Where the particular nucleotide occurrence of a SNP, or nucleotide occurrences of a haplotype, is such that the nucleotide occurrence results in an amino acid change in an encoded polypeptide, the nucleotide occurrence can be identified indirectly by detecting the particular amino acid in the polypeptide. The method for determining the amino acid will depend, for example, on the structure of the polypeptide or on the position of the amino acid in the polypeptide. Where the polypeptide contains only a single occurrence of an amino acid encoded by the particular SNP, the polypeptide can be examined for the presence or absence of the amino acid. For example, where the amino acid is at or near the amino terminus or the carboxy terminus of the polypeptide, simple sequencing of the terminal amino acids can be performed. Alternatively, the polypeptide can be treated with one or more enzymes and a peptide fragment containing the amino acid position of interest can be examined, for example, by sequencing the peptide, or by detecting a particular migration of the peptide following electrophoresis. Where the particular amino acid comprises an epitope of the polypeptide, the specific binding, or absence thereof, of an antibody specific for the epitope can be detected. Other methods for detecting a particular amino acid in a polypeptide or peptide fragment thereof are well known and can be selected based, for example, on convenience or availability of equipment such as a massspectrometer, capillary electrophoresis system, magnetic resonance imaging equipment, and the like.

A method for inferring the iris color trait of a human subject from a nucleic acid sample of the human subject by identifying a nucleotide occurrence in the sample for an SNP according to the invention can further include grouping the nucleotide occurrences of the SNPs for a gene or a particular area of the genome, such as the region of the HERC2 gene, into one marker allele or more haplotype alleles. The identified single marker / haplotype alleles then can be compared to known marker / haplotype alleles such that, when the relationship of the known marker / haplotype alleles to the eye color is known, an inference can be drawn as to the iris color of the subject providing the nucleic acid sample. Identification of the nucleotide occurrence can be performed using any method suitable for examining the particular sample. For example, wherein the sample contains nucleic acid molecules, the identification can be performed by contacting polynucleotides in (or derived from) the sample with a specific binding pair member that selectively hybridizes to a region of the polynucleotide that includes the SNP or SNPs, under conditions wherein the binding pair member specifically binds at or near the SNP(s). The binding pair member can be any molecule that specifically binds or associates with the target polynucleotide, including, for example, an antibody or an oligonucleotide.

Methods of the invention can in principle be performed by using any nucleic acid amplification method, such as the Polymerase Chain Reaction (PCR; Mullis 1987, U.S. Pat. No. 4,683,195, 4,683,202, en 4,800,159) or by using amplification reactions such as Ligase Chain Reaction (LCR; Barany 1991, Proc. Natl. Acad. Sci. USA 88:189-193; EP Appl. No., 320,308), Self-Sustained Sequence Replication (3SR; Guatelli et al., 1990, Proc. Natl. Acad. Sci. USA 87:1874-1878), Strand Displacement Amplification (SDA; U.S. Pat. Nos. 5,270,184, en 5,455,166), Transcriptional Amplification System (TAS; Kwoh et al., Proc. Natl. Acad. Sci. USA 86:1173-1177), Q-Beta Replicase (Lizardi et al., 1988, Bio/Technology 6:1197), Rolling Circle Amplification (RCA; U.S. Pat. No. 5,871,921), Nucleic Acid Sequence Based Amplification (NASBA), Cleavase Fragment Length Polymorphism (U.S. Pat. No. 5,719,028), Isothermal and Chimeric Primer-initiated Amplification of Nucleic Acid (ICAN), Ramification-extension Amplification Method (RAM; U.S. Pat. Nos. 5,719,028 and 5,942,391) or other suitable methods for amplification of DNA.

In order to amplify DNA with a small number of mismatches to one or more of the amplification primers, an Amplification reaction may be performed under conditions of reduced stringency (e.g. a PCR Amplification using an annealing temperature of 38°C, or the presence of 3.5 mM MgCl2). The person skilled in the art will be able to select conditions of suitable stringency.

The primers herein are selected to be "substantially" complementary (i.e. at least 65%, more preferably at least 80% perfectly complementary) to their target regions present on the different strands of each specific sequence to be amplified. It is possible to use primer sequences containing e.g. inositol residues or ambiguous bases or even primers that contain one or more mismatches when compared to the target sequence. In general, sequences that exhibit at least 65%, more preferably at least 80% homology with the target DNA oligonucleotide sequences, are considered suitable for use in a method of the present invention. Sequence mismatches are also not critical when using low stringency hybridization conditions.

The detection of the amplification products can in principle be accomplished by any suitable method known in the art. The detection fragments may be directly stained or labelled with radioactive labels, antibodies, luminescent dyes, fluorescent dyes, or enzyme reagents. Direct DNA stains include for example intercalating dyes such as acridine orange, ethidium bromide, ethidium monoazide or Hoechst dyes.

Alternatively, the DNA fragments may be detected by incorporation of labelled dNTP bases into the synthesized DNA fragments. Detection labels which may be associated with nucleotide bases include e.g. fluorescein, cyanine dye or BrdUrd.

When using a probe-based detection system, a suitable detection procedure for use in the present invention may for example comprise an enzyme immunoassay (EIA) format (Jacobs et al., 1997, J. Clin. Microbiol. 35, 791-795). For performing a detection by manner of the EIA procedure, either the forward or the reverse primer used in the amplification reaction may comprise a capturing group, such as a biotin group for immobilization of target DNA PCR amplicons on e.g. a streptavidin coated microtiter plate wells for subsequent EIA detection of target DNA -amplicons (see below). The skilled person will understand that other groups for immobilization of target DNA PCR amplicons in an EIA format may be employed.

Probes useful for the detection of the target DNA as disclosed herein preferably bind only to at least a part of the DNA sequence region as amplified by the DNA amplification procedure. Those of skill in the art can prepare suitable probes for detection based on the nucleotide sequence of the target DNA without undue experimentation as set out herein. Also the complementary sequences of the target DNA may suitably be used as detection probes in a method of the invention, provided that such a complementary strand is amplified in the amplification reaction employed.

Suitable detection procedures for use herein may for example comprise immobilization of the amplicons and probing the DNA sequences thereof by e.g. southern blotting. Other formats may comprise an EIA format as described above. To facilitate the detection of binding, the specific amplicon detection probes may comprise a label moiety such as a fluorophore, a chromophore, an enzyme or a radio-label, so as to facilitate monitoring of binding of the probes to the reaction product of the amplification reaction. Such labels are well-known to those skilled in the art and include, for example, fluorescein isothiocyanate (FITC), β-galactosidase, horseradish peroxidase, streptavidin, biotin, digoxigenin, 35S or 125I. Other examples will be apparent to those skilled in the art.

Detection may also be performed by a so called reverse line blot (RLB) assay, such as for instance described by Van den Brule et al. (2002, J. Clin. Microbiol. 40, 779-787). For this purpose RLB probes are preferably synthesized with a 5'amino group for subsequent immobilization on e.g. carboxyl-coated nylon membranes. The advantage of an RLB format is the ease of the system and its speed, thus allowing for high throughput sample processing.

Any suitable method for screening the nucleic acids for the presence or absence of polymorphisms is considered to be part of the instant invention. Such methods include, but are not limited to: DNA sequencing, restriction fragment length polymorphism (RFLP) analysis, amplified fragment length polymorphism (AFLP) analysis; heteroduplex analysis, single strand conformational polymorphism (SSCP) analysis, denaturing gradient gel electrophoresis (DGGE), real time PCR analysis (e.g. Taqman®), temperature gradient gel electrophoresis (TGGE), primer extension, allele-specific hybridization, and INVADER® genetic analysis assays, cleavase fragment length polymorphism (CFLP) analysis, sequence-characterized amplified region (SCAR) analysis, cleaved amplified polymorphic sequence (CAPS) analysis

The use of nucleic acid probes for the detection of specific DNA sequences is well known in the art. Mostly these procedures comprise the hybridization of the target DNA with the probe followed by post-hybridization washings. Specificity is typically the function of post-hybridization washes, the critical factors being the ionic strength and temperature of the final wash solution. For DNA-DNA hybrids, the Tm can be approximated from the equation of Meinkoth and Wahl, Anal. Biochem., 138: 267-284 (1984): Tm = 81.5 °C + 16.6 (log M) + 0.41 (% GC)-0.61 (% form)-500/L; where M is the molarity of monovalent cations, % GC is the percentage of guanosine and cytosine nucleotides in the DNA, % form is the percentage of formamide in the hybridization solution, and L is the length of the hybrid in base pairs. The Tm is the temperature (under defined ionic strength and pH) at which 50% of a complementary target sequence hybridizes to a perfectly matched probe. Tm is reduced by about 1°C for each 1 % of mismatching; thus, the hybridization and/or wash conditions can be adjusted to hybridize to sequences of the desired identity. For example, if sequences with > 90% identity are sought, the Tm can be decreased 10°C. Generally, stringent conditions are selected to be about 5 C lower than the thermal melting point (Tm) for the specific sequence and its complement at a defined ionic strength and pH. However, severely stringent conditions can utilize a hybridization and/or wash at 1,2,3, or 4 °C lower than the thermal melting point (Tm); moderately stringent conditions can utilize a hybridization and/or wash at 6,7,8,9, or 10 °C lower than the thermal melting point (Tm); low stringency conditions can utilize a hybridization and/or wash at 11,12,13,14,15, or 20°C lower than the thermal melting point (Tm). Using the equation, hybridization and wash compositions, and desired Tm, those of ordinary skill will understand that variations in the stringency of hybridization and/or wash solutions are inherently described. If the desired degree of mismatching results in a Tm of less than 45 °C (aqueous solution) or 32 °C (formamide solution) it is preferred to increase the SSC concentration so that a higher temperature can be used. An extensive guide to the hybridization of nucleic acids is found in Tijssen, Laboratory Techniques in Biochemistry and Molecular Biology-Hvbridization with Nucleic Acid Probes, Part I, Chapter 2 "Overview of principles of hybridization and the strategy of nucleic acid probe assays", Elsevier. New York (1993); and Current Protocols in Molecular Biology, Chapter 2, Ausubel, et al., Eds., *supra.*

The development of primers and probes useful for the detection of polymorphic positions in a nucleic acid is within the realm of ordinary skill (see for instance Sambrook, J., Russell D.W., Sambrook, J. (2001) Molecular Cloning: a Laboratory Manual. Cold Spring Harbor Laboratory Press, Plainview, N.Y.).

By using standard DNA technology it is possible to produce probes and primers that directly or indirectly hybridize to the DNA samples to be tested or cDNA produced from RNA by reverse transcription, and which can be used in assays for the detection of the SNPs. Nucleic acid amplification techniques allow the amplification of fragments of nucleic acids, which may be present in very low amounts.

In order to develop nucleic acid-based detection methods, the SNP-specific sequences must be determined for which primers or probes may then be developed. To detect the SNPs by nucleic acid amplification and/or probe hybridization, the nucleic acid may be isolated from any raw sample material, optionally reverse transcribed into cDNA and directly cloned and/or sequenced. DNA and RNA isolation kits are commercially available from for instance QIAGEN GmbH, Hilden, Germany, or Roche Diagnostics, a division of F. Hoffmann-La Roche Ltd, Basel, Switzerland.

A sample useful for practicing a method of the invention can be any biological sample of a subject that contains nucleic acid molecules, including portions of the gene sequences to be examined, or corresponding encoded polypeptides, depending on the particular method. As such, the sample can be a cell, tissue or organ sample, or can be a sample of a biological fluid such as semen, saliva, blood, and the like. A nucleic acid sample useful for practicing a method of the invention will depend, in part, on whether the SNPs of the haplotype to be identified are in coding regions or in non-coding regions. Thus, where at least one of the SNPs to be identified is in a noncoding region, the nucleic acid sample generally is a deoxyribonucleic acid (DNA) sample, particularly genomic DNA or an amplification product thereof. However, where heteronuclear ribonucleic acid RNA), which includes unspliced mRNA precursor RNA molecules, is available, a cDNA or amplification product thereof can be used. Where each of the SNPs of the haplotype is present in a coding region of the pigmentation gene (s), the nucleic acid sample can be DNA or RNA, or products derived therefrom, for example, amplification products. Furthermore, while the methods of the invention generally are exemplified with respect to a nucleic acid sample, it will be recognized that particular haplotype alleles can be in coding regions of a gene and can result in polypeptides containing different amino acids at the positions corresponding to the SNPs due to non-degenerate codon changes. As such, in another aspect, the methods of the invention can be practiced using a sample containing polypeptides of the subject.

Methods of the invention can be practiced with respect to human subjects and, therefore, can be particularly useful for forensic analysis. In a forensic application or a method of the invention, the human nucleic acid sample can be obtained from a crime scene, using well established sampling methods. Thus, the sample can be fluid sample or a swab sample for example blood stain, semen stain, hair follicle, or other biological specimen, taken from a crime scene, or can be a soil sample suspected of containing biological material of a potential crime victim or perpetrator, can be material retrieved from under the finger nails of a potential crime victim, or the like, wherein nucleic acids (or polypeptides) in the sample can be used as a basis for drawing an inference as to a iris color according to a method of the invention. Another application of the invention is in identifying missing persons by analysing the herein identified markers from nucleic acids / proteins in body parts or samples from the whole body of the unknown person to be identified.

Using either the cloned nucleic acid as a hybridization probe, using sequence information derived from the clone, or by designing degenerative primers based on the sequence of the SNP and its flanking sequences, nucleic acid hybridization probes and/or nucleic acid amplification primers may be designed an used in a detection assay for detecting the characteristics of the one or more SNPs in a sample as defined herein.

The DNA, or alternatively, the cDNA may be PCR amplified by using for instance Pfu and Taq DNA polymerases and amplification primers specific for the SNP DNA sequences. Also complete commercially available systems may be used for PCR (e.g. available form various suppliers such as Roche Diagnostics). A suitable method may for instance include mixing into a suitable aqueous buffering system (e.g. a commercially available PCR buffer) a suitable amount of total DNA as a template (e.g. 1 to 5 µg), a suitable amount (e.g. 10 pmol) of a pair of bi-directional amplification primers, a suitable amount of dNTPs and the DNA polymerase, denaturing the nucleic acids by boiling for 1 min, and performing a cycling reaction of around 10-50 alternating cycles of stringent primer hybridization, strand elongation and denaturing, at suitable temperatures to obtain DNA copies of the DNA template as amplification product. The amount of copies produced upon a certain number of cycles correlates directly to the amount of target DNA in the DNA template.

The skilled person is well aware of the available quantitative PCR methods presently available from commercial suppliers to quantify the amount of target DNA in the template. The term "hybridization signal" as used herein inter alia refers to the amount of amplification product produced upon a certain number of cycles and thus to the amount of target DNA available as template in the reaction.

In order to amplify a nucleic acid with a small number of mismatches to one or more of the amplification primers, an amplification reaction may be performed under conditions of reduced stringency (e.g. a PCR amplification using an annealing temperature of 38°C, or the presence of 3.5 mM MgCl2). The person skilled in the art will be able to select conditions of suitable stringency.

The primers herein are selected to be "substantially" complementary (i.e. at least 65%, more preferably at least 80% perfectly complementary) to their target regions present on the different strands of each specific sequence to be amplified. It is possible to use primer sequences containing e.g. inositol residues or ambiguous bases or even primers that contain one or more mismatches when compared to the target sequence. In general, sequences that exhibit at least 65%, more preferably at least 80% homology with the target DNA or RNA oligonucleotide sequences are considered suitable for use in a method of the present invention. Sequence mismatches are also not critical when using low stringency hybridization conditions.

The detection of the amplification products can in principle be accomplished by any suitable method known in the art. The amplified fragments may be directly stained or labelled with radioactive labels, antibodies, luminescent dyes, fluorescent dyes, or enzyme reagents. Direct DNA stains include for example intercalating dyes such as acridine orange, ethidium bromide, ethidium monoazide or Hoechst dyes.

Alternatively, the DNA or RNA fragments may be detected by incorporation of labelled dNTP bases into the synthesized fragments. Detection labels which may be associated with nucleotide bases include e.g. fluorescein, cyanine dye, digoxigenin (DIG) or bromodeoxyuridine (BrdUrd).

In a quantitative PCR method, the reaction is preferably performed by using an oligonucleotide primer that contains one or more 'locked' nucleic acid (LNA®) monomers, or by using LNA® fluorescent probes. LNA® technology involves an oligonucleotide (probe or primer that contains one or more LNA® monomers [2'-O, 4'-C-methylene-B-D-ribofuranosyl-modified] (e.g. Petersen and Wengel, 2003. TRENDS in Biotechnology Vol.21(2):74-81). In an LNA monomer, the ribose sugar moiety of the nucleotide is modified, while the base itself is unaltered. The result is a covalent bridge that 'locks' the ribose in the N-type (3'-endo) conformation, which enhances base stacking and phosphate backbone pre-organisation. This provides the oligonucleotide with improved affinity for complementary DNA or RNA sequences and therefore a higher Tm. When using LNA® primers, the detection of the double stranded amplification products may for instance be performed by using a double-stranded DNA stain, such as SYBR Green® [Molecular Probes, Inc.] (see for instance Ponchel et al. 2003, BMC Biotechnology 3:18).

Other methods of analysing the nuclei acid suitably comprise the use of a primer extension assay; a Taqman® PCR; a differential hybridization assay; an assay which detects allele-specific enzyme cleavage; and/or allele-specific PCR.

When using a probe-based detection system, a suitable detection procedure for use in the present invention may for example comprise an enzyme immunoassay (EIA) format (Jacobs et al., 1997, J Clin Microbiol 35:791-795). For performing a detection by manner of the EIA procedure, either the forward or the reverse primer used in the amplification reaction may comprise a capturing group, such as a biotin group for immobilization of target DNA PCR amplicons on e.g. a streptavidin coated microtiter plate wells or streptavidin coated Dynabeads® (Dynal Biotech, Oslo, Norway) for subsequent EIA detection of target DNA-amplicons. The skilled person will understand that other groups for immobilization of target DNA PCR amplicons in an EIA format may be employed.

Probes useful for the detection of the target nucleic acid sequences as disclosed herein preferably bind only to at least a part of the nucleic acid sequence region as amplified by the nucleic acid amplification procedure. Those of skill in the art can prepare suitable probes for detection based on the nucleotide sequence of the target nucleic acid without undue experimentation as set out herein. Also the complementary nucleotide sequences, whether DNA or RNA or chemically synthesized analogues, of the target nucleic acid may suitably be used as type-specific detection probes in a method of the invention, provided that such a complementary strand is amplified in the amplification reaction employed.

Suitable detection procedures for use herein may for example comprise immobilization of the amplicons and probing the nucleic acid sequences thereof by e.g. Northern and Southern blotting. Other formats may comprise an EIA format as described above. To facilitate the detection of binding, the specific amplicon detection probes may comprise a label moiety such as a fluorophore, a chromophore, an enzyme or a radio-label, so as to facilitate monitoring of binding of the probes to the reaction product of the amplification reaction. Such labels are well known to those skilled in the art and include, for example, fluorescein isothiocyanate (FITC), β-galactosidase, horseradish peroxidase, streptavidin, biotin, digoxigenin, 35S, 14C, 32P or 125I. Other examples will be apparent to those skilled in the art.

Detection may also be performed by a so-called reverse line blot (RLB) assay, such as for instance described by Van den Brule et al. (2002). For this purpose RLB probes are preferably synthesized with a 5' amino group for subsequent immobilization on e.g. carboxyl-coated nylon membranes. The advantage of an RLB format is the ease of the system and its speed, thus allowing for high throughput sample processing.

The use of nucleic acid probes for the detection of RNA or DNA fragments is well known in the art. Mostly these procedures comprise the hybridization of the target nucleic acid with the probe followed by post-hybridization washings. Specificity is typically the function of post-hybridization washes, the critical factors being the ionic strength and temperature of the final wash solution. For nucleic acid hybrids, the Tm can be approximated from the equation of Meinkoth and Wahl (1984): Tm = 81.5 °C + 16.6 (log M) + 0.41 (% GC)-0.61 (% form)-500/L; where M is the molarity of monovalent cations, % GC is the percentage of guanosine and cytosine nucleotides in the nucleic acid, % form is the percentage of formamide in the hybridization solution, and L is the length of the hybrid in base pairs. The Tm is the temperature (under defined ionic strength and pH) at which 50% of a complementary target sequence hybridizes to a perfectly matched probe. Tm is reduced by about 1 °C for each 1 % of mismatching; thus, the hybridization and/or wash conditions can be adjusted to hybridize to sequences of the desired identity. For example, if sequences with > 90% identity are sought, the Tm can be decreased 10°C. Generally, stringent conditions are selected to be about 5 °C lower than the thermal melting point (Tm) for the specific sequence and its complement at a defined ionic strength and pH. However, severely stringent conditions can utilize a hybridization and/or wash at 1,2,3, or 4 °C lower than the thermal melting point (Tm); moderately stringent conditions can utilize a hybridization and/or wash at 6, 7, 8, 9, or 10 °C lower than the thermal melting point (Tm); low stringency conditions can utilize a hybridization and/or wash at 11, 12, 13, 14, 15, or 20 °C lower than the thermal melting point (Tm). Using the equation, hybridization and wash compositions, and desired Tm, those of ordinary skill will understand that variations in the stringency of hybridization and/or wash solutions are inherently described. If the desired degree of mismatching results in a Tm of less than 45 °C (aqueous solution) or 32 °C (formamide solution) it is preferred to increase the SSC concentration so that a higher temperature can be used. An extensive guide to the hybridization of nucleic acids is found in Tijssen, 1993 supra; Ausubel et al., 1998 supra.

In another aspect, the invention provides oligonucleotide probes for the detection of the SNP. The detection probes herein are selected to be "substantially" complementary to a single stranded nucleic acid molecule, or to one of the strands of the double stranded nucleic acids generated by an amplification reaction of the invention. Preferably the probes are substantially complementary to the, optionally immobilized (e.g. biotin labelled) antisense strands of the amplicons generated from the target RNA or DNA.

It is allowable for detection probes of the present invention to contain one or more mismatches to their target sequence. In general, sequences that exhibit at least 65%, more preferably at least 80% homology with the target oligonucleotide sequences are considered suitable for use in a method of the present invention.

The invention will now be illustrated by way of the following, non limiting Example.

### EXAMPLES

### MATERIAL AND METHODS

### Study populations

### The ERF study population

For the ERF study, all living descendants and spouses of 22 couples living in the Rucphen region of southwest Netherlands in the 19th century were invited. These couples parented a minimum of six children and their genealogical relationships are known up to the middle of the 18th century when the population was founded by approximately 150 individuals. The population now includes approximately 20,000 residents who show an increased linkage disequilibrium compared to outbred populations (Pardo et al., 2005; Service et al., 2006).

The Medical Ethics Committee of the Erasmus Medical Center approved the study protocol, and all participants provided written informed consent. Information on iris color was collected for all participants and genomic DNA was extracted from peripheral venous blood utilizing the salting out method (Miller et al. 1988). For the initial GWA screen, we selected 192 distantly related (≥ 5 generations) individuals from the ERF population (Table 1). Individuals were initially selected for a study of height but keeping with the assumption that the genes involved in human height and iris color are independently transmitted we used the GWA data for the current project. GWA and iris color data were available for 192 participants and replication studies were conducted using 2217 ERF participants for whom data on iris color was available (Table 1). For genome-wide linkage analysis we used 1292 ERF individuals that had iris color data and were genotyped for the Illumina 6K linkage panel. In addition, we used 733 ERF participants that had iris color data and were genotyped for the Illumina 300 Duo SNP panel.

### The Rotterdam study population

The Rotterdam Study is a population-based prospective cohort study in 7,983 subjects aged 55 years and older residing in Ommoord, a suburb of Rotterdam, that aims to assess the occurrence and determinants of chronic diseases in later life (Hofman et al. 1991). This is an outbred population, mostly of Dutch European origin. In 1990-1993, the participants were invited to visit the research center for a clinical examination. The Medical Ethics Committee of the Erasmus Medical Center approved the study protocol, and all participants provided written informed consent. Information on iris color was collected for all participants and genomic DNA was extracted from peripheral venous blood utilizing the salting out method (Miller et al. 1988). We selected 509 unrelated women aged between 60 and 75 years for an independent GWA screen (Table 1). Women using medication for hypertension, diabetes or lipid-lowering drugs and hormone replacement therapy during follow-up were excluded, as well as any remaining women with history of incident/prevalent myocardial infarction, stroke, cancer or hip fractures. Although these women were selected for a pilot GWA study for osteoporosis and other disorders, we assumed that iris color was segregating independently from these traits. For 481 women, GWA and data on iris color were available (Table 1) and for replication studies DNA was available for 6056 participants with known iris color phenotypes (Table 1).

### Phenotype collection

In both studies, each eye was examined by slit lamp examination and iris color was graded using standard images showing various degrees of iris pigmentation. Three categories of iris color (blue, intermediate, and brown) were distinguished based on predominant color and the amount of yellow or brown pigment present in the iris. Iris color phenotypes were summarized in Table 1. Differences between blue and brown iris color frequencies between the ERF study and the Rotterdam study can most likely be explained by the influence of the Spanish occupation of the region in the 16th and 17th century, which lasted for almost hundred years in the mostly southern parts of Netherlands.

### Genotype collection

### Microarray genotyping

For the Rotterdam study the GeneChip® Human M apping 500K Array Set (Affymetrix) was utilized. Because of the marked linkage disequilibrium observed previously in ERF (Aulchenko et al., 2004; Service et al., 2006) we applied the 250K Nsp array from the GeneChip® Human Mapping 500K Array Set (Affymetrix) in the ERF study. Microarray based genotyping was performed at Erasmus MC according to the manufacturer's instructions. Markers were excluded if they deviated significantly from Hardy-Weinberg equilibrium (P<0.001), if they had low minor allele frequency (MAF<0.025), or if they had a call rate <95% in all samples. Further we excluded 10 women from the Rotterdam pilot study who did not cluster with the otherwise homogeneous sample containing 98% of the participants (pairwise population concordance test < 0.0001) (Purcell et al. 2007). For the linkage analysis in the ERF cohort we used the Illumina Infinium Linkage assay. This panel includes 5,861 markers distributed evenly across the human genome (median distance between the marker 301 kb). We additional used for GWA the Illumina 300 Duo product containing ~317000 tagging SNPs across the human genome. Illumina genotyping was performed at the Centre National de Génotypage in France according to the manufacturer's instruction.

### TaqMan genotyping

For conformation studies four SNPs were genotyped in the total ERF (N=2217) and the total Rotterdam (N=6056) study populations using TaqMan technology with rs11855019, rs7495174, and rs6497268 typed using Custom Taqman assays (Applied Biosystems) and rs916977 using the Taqman genotyping assay C_2567831_10 (Applied Biosystems). Primer and probe sequences of the first three SNPs are available on request from the authors, whereas the assay for rs916977 is commercially available from the manufacturer. 1-2 ng genomic DNA was dispensed into 384-wells plates using a Caliper Sciclone ALH3000 pipetting robot (Caliper LS). All assays were run in a total volume of 2 µl using 2-5 ng of genomic DNA, 0.025-0.05 µl of 40x assay mix, and 1 µl ABSOLUTE QPCR mix (ABgene) or Taqman Universal PCR Master Mix (Applied Biosystems). Reagents were dispensed in a 384-well plate using the Deerac Equator NS808 (Deerac Fluidics). PCR programs were 95C 15 min, 95C 15 sec and 60C 1 min for 40 cycles (ABSOLUTE QPCR Mix) or 94C 10 min, 94C 15 sec, 60C 1 min for 40 cycles (Taqman Universal PCR Master Mix) on Dual 384-well GeneAmp PCR system 9700 (Applied Biosystems) with subsequent end-point-reading on ABI 7900HT Real-Time PCR System (Applied Biosystems).

### Statistical analyses

### Association and linkage analysis

To test for associations using large amounts of SNPs we used fast functions implemented in R library GenABEL version 1.1-8 (Aulchenko et al. 2007). In the GWA analysis, p-values were estimated using the Armitage's trend test in 2 by 3 contingency tables (blue and nonblue iris color against three possible genotypes) with 1 degree of freedom. In the replication analyses of SNPs of interest and based on the enlarged populations, odds ratios (ORs) were derived using multiple logistic regressions using a binary outcome (brown and non-brown color). To adjust for multiple testing with a large number of correlated markers, significance was also estimated against the empirical distribution of the chi-square statistics after 1000 genome-wide permutations. We used the genomic control method (Devlin and Roeder 1999) to adjust for the relationship between ERF participants (Steinthorsdottir et al., 2007). Based on the Illumina Infinium Linkage Assay the inflation factor was estimated to be 1.12 for the ERF study. No inflation of test statistics was observed in Rotterdam study (Lambda=1.00), using the Affymetrix GeneChip® Human M apping 500K Array. Regions flanking significant SNPs in either GWA screens were further investigated using haplotype analysis. Two methods were used: (1) sliding window of 2 and 3 continuous SNPs using the R library haplo.stats version 1.3 (Schaid et al. 2002) and (2) haplotype block analysis using the software package Haploview version 3.32, where the blocks were defined using 95% confidence bounds of D' (Gabriel et al., 2002). Power calculations using a genomewide type-I error rate of 5% shows that the probability is 80% to identify as part of the GWA a SNP explaining 18% of phenotypic variance for the ERF study to detect a SNP while for the Rotterdam Study the power was 80% to detect a SNP explaining 8% of the variance as part of the GWA. For the ERF study, using a genome-wide type-I error rate of 5% the probability is 80% to detect linkage with a variant explaining 2-3% of phenotypic variance. For the linkage analysis in ERF we used the variance-component models as implemented in the SOLAR (Sequential Oligogenic Linkage Analysis Routines) computer package (Almasy and Blangero 1998). Prior to analysis, ERF genealogy was split to 18-bits pedigrees using PedSTR software. The genomic identity-by-descent (IBD) was estimated using the MERLIN software package (Abecasis et al. 2002). Cutting complex pedigrees may lead to false positive linkage because true kinship is under-estimated. Therefore instead of pedigree kinship estimated from the split 18-bit pedigrees, we estimated null kinship as an average of marker IBD across the genome.

### Analyses of predictive value

To investigate the value of the SNPs identified for the prediction of iris color, we constructed a prediction model in the Rotterdam GWA pilot sample and in a random 50% sample of the Rotterdam study (derivation dataset) using logistic regression analysis.

Separate models were constructed for brown and blue iris color (yes/no). The model was validated in the other 50% of the Rotterdam study (internal validation) and in the ERF study (external validation). For each individual we calculated the probabilities of brown and blue irides. The predictive value was assessed by the area under the receiver operating characteristic curve (AUC), which is a measure of discriminative accuracy indicating the degree to which the predicted probabilities can discriminate between individuals with brown (or blue) irides and those without. AUC ranges from 0.5 representing total lack of discrimination to 1.0 representing perfect discrimination (Hanley and McNeil 1982).

### Spatial autocorrelation and Pearson's correlation analysis

Allele frequencies of rs916977 were obtained from genome-wide SNP data of 23 European populations (of which the Rotterdam study is one) as part of a different project. The spatial pattern of the allelic frequencies of the SNP rs916977 was analysed by means of spatial autocorrelation (Sokal and Oden, 1978) using the PASSAGE program. This method plots the amount of autocorrelation (expressed as Moran's I index) between pairs of populations against their geographic distance. For correlation analysis, European iris color frequency distribution was obtained from a map published elsewhere (Beals and Hoijer 1965) and the mean values of iris color frequency classes were assigned to each of the 23 population studied based on their geographic origin. A Pearson's correlation was performed between this inferred value of iris color phenotypes and the frequency of the C allele of rs916977 in each population.

### RESULTS

### Findings of the association and linkage studies

In the GWA analysis (250 K SNPs) of the 192 unrelated individuals from the ERF study, rs916977 located at 26.2Mb of chromosome 15 reached genome-wide significance (nominal P-value = 1.18×10-9, empirical genome-wide P-value adjusted for multiple testing < 0.001, Figures 1A, 2A). This marker was located in the intron 11 of the *HERC2* gene.

Seven additional SNPs flanking rs916977 reached nominal significance of < 0.01 (rs16950821 and rs8024968 in the OCA2 gene; rs6497287, rs8041209, rs8028689, rs2346050 in the *HERC2* gene; rs4966231 5' proximal of *HERC2;* see figure 2A for the single SNP and sliding window haplotype analysis). This region covered the 5' proximal part of the *OCA2* gene and the complete *HERC2* gene up to 5'proximal *of HERC2.* In the additionally obtained Illumina 300 Duo data from 733 ERF participants we found 18 SNPs with significant iris color association on the genome-wide level (P<0.01) of which 8 are in the *HERC2* gene (rs7183877, rs2240204, rs8039195, rs16950979, rs16950987, rs1667394, rs1635168, and rs8028689) and 10 are in the OCA2 gene (rs2594935, rs728405, rs3794604, rs4778232, rs8024968, rs1597196, rs7179994, rs4778138, rs4778241, and rs7495174) (Table 5). Also, the genome-wide linkage analysis in 1457 participants of the ERF study using the 6K Illumina linkage panel gave very convincing evidence for linkage of iris color to chromosome 15q13.1. The highest LOD score obtained across the genome was 29.47 at rs4778137 from intron 1 of the *OCA2* gene; this was the only peak in the linkage analysis reaching a LOD score over 3 (see Figure 3).

Also the second, independent GWA (500 K SNPs) in 481 women from the Rotterdam study identified the same region 15q13.1 as most strongly associated with iris color. Again, rs916977 in the HERC2 gene showed the strongest association with iris color (nominal Pvalue = 2.08×10-40, genome-wide empirical P-value < 0.001). In addition, seven other *HERC2* SNPs flanking rs916977 in the 26.1-26.2 Mb region (rs6497287, rs8041209, rs8028689, rs2346050, rs6497292, rs2240202, rs12592730 of which the latter three were not included in the 250K screen in the ERF study) reached genome-wide significance (Pvalue < 0.001, nominal p-values of 10-13 to 10-19, Figure 1b and 2b). In addition, four intronic SNPs (rs3930739, rs1448485, rs16950821, rs8024968) from *OCA2* showed nominal p-values <10-5 in the Rotterdam study (Figure 2B). No other marker outside the *HERC2* gene revealed genome-wide significant association to iris color in the Rotterdam study using the P<0.001 level. One region on chromosome 9 showed borderline genome-wide significance on the 0.5% level but was not confirmed the ERF association or linkage study.

To further investigate the 15q13.1 region we examined the haplotype block structure and linkage disequilibrium pattern in ERF and the Rotterdam study (Gabriel et al. 2002). The linkage disequilibrium pattern was similar in the ERF and Rotterdam study. Three blocks were identified in both populations where the 3^{rd} block contains the *HERC2* SNP rs916977 showing the strongest association in both populations (ERF study: P-value for block 3 = 5.94 or 3×10-6, Rotterdam study: P-value = 8.93 or 8.39 ×10-20 (Table 2). The 2^{nd} and the 3^{rd} block were not in strong linkage disequilibrium in both populations (ERF study: r2 =0.40; Rotterdam study: r2=0.39). The 2^{nd} block is physically closer to intron 1 of the *OCA2* gene, the region that was previously reported to be the most important region for iris color (Duffy et al., 2007). This block also showed some evidence for association with iris color in the Rotterdam study, but notably at a much lower significance level compared to the 3rd block (nominal P-value for the block=7.61×10-3). The second block was only marginally significant in the ERF study (nominal P-value block 2 =0.01) (Table 2). Also block 1 showed some evidence for association to iris color in the Rotterdam study but this is not confirmed in the ERF study. Neither block 1 nor block 2 reached genome-wide significance when adjusting for multiple testing. A common haplotype of 5 SNPs from the 3^{rd} block in the Rotterdam study, CTCGA, was found more often in people with blue iris color (97%) than in those with brown iris color (58.7%, intermediate iris color: 76.5%) (Table 2). The same haplotype is found to be associated with iris color in ERF (with rs4932620 missing which was not present in the 250k array) with a frequency of 96.8% in those with blue iris color compared with 65.7% in brown ones (intermediates: 91.9%). Two additional haplotypes are found in ERF and ERGO and are associated with non-blue iris color (Table 2).

### SNP typing of the OCA2-HERC2 region in the total ERF and Rotterdam studies

To confirm the GWA finding we typed rs916977 in *HERC2* in all 2217 ERF study participants and in all 6056 Rotterdam study participants available (Table 1). Further, we genotyped the three SNPs in intron 1 of the *OCA2* gene (rs11855019, rs6497268, and rs7495174) that were recently reported to be associated strongest with iris color (Duffy et al., 2007) but were not part of the Affymetrix SNP arrays we used. SNP rs916977 is located about 168kb 5' proximal of the three SNPs from intron 1 of the *OCA2* gene. The latter region also showed the highest LOD score in our linkage analysis of the ERF study. When analysed separately and ignoring linkage disequilibrium, all four SNPs were strongly associated with iris color in both enlarged populations (p<5.0x10-76; see Table 3). However, SNP rs916977 from the *HERC2* gene displayed the highest statistically significance (1.9×10-113 for ERF and <1.0×10-300 for Rotterdam study). When including all four SNPs together in the regression model, rs916977 (ERF: 2.02x10-33; Rotterdam study: p < 5.84x10-113), rs11855019 (ERF: 2.63x10-9; Rotterdam study: 6.75x10-6), and rs7495174 (ERF: 9.71x10-9; Rotterdam study: 5.09x10-9) remained significantly associated to iris color, with rs916977 clearly being the most informative marker of the four tested. The haplotype analysis based on these four SNPs in the two enlarged populations showed that the TGTC haplotype (rs11855019, rs6497268, rs7495174, and rs916977) was found in 75.5% of the ERF and in 81.2% of the Rotterdam study participants. This haplotype was mostly found in individuals with blue irides, and in significantly lower frequency in those with brown and intermediate iris color (ERF study: 92.0%, 55.6%, and 79.4%; Rotterdam study: 91.2%, 55.3%, and 72.1%) (Table 4). We observed 10 additional haplotypes in the ERF and the Rotterdam study. All of these were found in a higher frequency in individuals with brown iris color compared with those with blue iris color (except for the haplotype CTTC).

### Iris color prediction based on SNP genotypes

First, we evaluated the predictive value of the *HERC2* rs916977, the SNP replicated in the ERF (GWA and linkage) and the Rotterdam study (GWA), together with the three SNPs in intron 1 of the *OCA2* gene (rs11855019, rs6497268, and rs7495174) identified by Duffy et al. (Duffy et al., 2007) and subsequently confirmed in the ERF and Rotterdam study. Figure 5a shows the discriminative accuracy for the prediction of brown iris color in the Rotterdam and ERF study data based on the four SNPs. The AUC for the prediction of brown iris color was 0.81 in the derivation dataset, 0.81 in the internal validation dataset of the Rotterdam study and 0.79 in the total ERF population. For blue iris color these values were slightly lower but comparable (0.80, 0.80 and 0.76; data not shown). The sharp angle in the ROC curves suggests a dominant role for one of the SNPs, which was rs916977. Figure 5b compares prediction of brown iris color based on all four SNPs with that based for the most strongly associated SNP, rs916977, by itself. Noteworthy, the AUC for the prediction of brown iris color based solely on rs916977 was 0.78 in the Rotterdam study and 0.71 in the ERF study. Based on the Rotterdam study, the predicted probability of brown iris color was 10.3% for homozygous carriers of the major rs916977 C-allele, 63.3% for heterozygotes and 84.7% for non-carriers. Although none of the genes previously implicated with iris color such *MATP, ASIP, TYRP1, CYP1A2, CYP2C8, and CYP2C9* (Kanetsky et al., 2002; Frudakis et al., 2003; Graf et al., 2005) reached genome wide significance in our two independent GWA studies, nor showed they convicing evidence for linkage in the ERF study, we did evaluate all SNPs covering those genes in our data set from the ERF and Rotterdam study. The only gene that showed consistent evidence of association to iris color in the ERF and the Rotterdam study was *TYRP1.* There were 8 consecutive SNPs (rs668603, rs497500, rs619678, rs650283, rs678535, rs500021, rs965209, and rs7857363) that showed nominal significant evidence for association (0.01<p<0.02). Adding these SNPs to the prediction model in the Rotterdam sample (n=491) did improve the predictive value marginally (AUC-increased from 0.81 to 0.84).

In our final model, we added four additional *OCA2* SNPs (rs768547, rs3751651, rs1448488, and rs730502), which we obtained from multiple backward regression analysis (P<0.02) when adjusting for the effect of all 41 SNPs available from the OCA2 gene in the Rotterdam study (after quality control). Adding these four SNPs to the model including rs916977 from *HERC2,* the three SNPs from intron 1 in OCA2, and the 8 SNPs from *TYRP1* improved the AUC to 0.87. In all analysis rs916977 from *HERC2* remained the SNP driving the predictive value.

### Spatial distribution of rs916977 alleles and correlation with iris color across Europe

An inspection of the allele frequencies of rs916977 in 23 populations of European-wide distribution (of which the Rotterdam study was one) showed that the C-allele, associated with blue iris color in the two GWA studies, was most frequent in northern Europe (the region where blue iris color is the major trait), and the T-allele, associated with brown iris color in the two GWA studies, was more frequent in southern Europe (the region where brown iris color is the major trait) (Figure 6A). A spatial autocorrelation analysis revealed that the allele frequencies of rs916977 in the 23 European populations studied followed a statistically significant clinal distribution across Europe (Figure 6B). In Figure 6A we superimposed the allele frequencies of rs916977 in the 23 European populations studied on the distribution of iris color phenotypes across Europe. As evident, there is a clear relationship between the distribution of the C-allele with that of light iris color and reverse the distribution of the T-allele with that of dark iris color in Europe. Note the highest frequency of rs916977C in Finland and Sweden that also showed the highest frequency of light iris color (>80%). To formally test this relationship we inferred the iris color phenotypes for the 23 populations studied from the iris color classes depicted in the figure (unfortunately, as far as we know the raw data for this figure were never published) and revealed a highly positive correlation between the allele distribution of rs916977 and the distribution of inferred iris color phenotypes (Pearson's r=0.7).

To conclude, we identified the intronic SNP rs916977 from the *HERC2* gene as the most significantly associated marker for human iris color variation on a genome-wide level in two independent population samples from The Netherlands and as marker with highest prediction value for iris color. Our findings suggest that genetic variants regulating expression of the *OCA2* gene exist in the *HERC2* gene or, alternatively, within the short sequence between the *OCA2* and *HERC2* genes, causing iris color variation in humans.

Our data also suggest that the genetic markers proposed here, rs916977 (and any closely linked marker in particular), are of value for the prediction of eye color in unknown persons for forensic applications of human identification. This is certainly applicable for individuals of European origin. Finally, it needs to be emphasized that any application of genetic markers for prediction of iris color or other visible traits for forensic purposes requires a clear legal basis, which does exists in The Netherlands.

**Table 1 Chiractetistics of the study populations**

| | Population size, N (%) | Blue | Iris color frequency Intermediate | Brown |
|---|---|---|---|---|
| *Independent GWA* screens | | | | |
| Population-based sample of ERF study | 192 | 4D.6 | 16.2 | 43.2 |
| Women | 95 (49.5) | 39.0 | 16.8 | 44.2 |
| Men | 97 (50.5) | 42.3 | 15.4 | 42.3 |
| Mean age, years (SD) | | 31.7 (6.6) | 32.5 (4.9) | 30.2 (6.9) |
| Population-based sample of Rotterdam study | 481 | 67.3 | 10.0 | 22.7 |
| Women | 481 (100) | 67.3 | 10.0 | 22.7 |
| Men | 0 | NA | NA | NA |
| Mean age, years (SD) | | 68.9 (3.7) | 68.1 (4.1) | 67.8 (3.8) |

| *Replication of candidate regions* | | | | |
|---|---|---|---|---|
| Total familly-based ERF study | 2217 | 41.1 | 20.9 | 38.0 |
| Women | 1248 (56.3) | 38.4 | 21.6 | 40.0 |
| Men | 969 (43.7) | 44.6 | 19.9 | 35.5 |
| Mean age, years.(SD) | | 49.7 (14.5) | 49.5 (13.9) | 47.7 (14.1) |
| Total population-based Rotterdam study | 6056 | 67.6 | 9.7 | 22.7 |
| Women | 3565 (58.9) | 66.6 | 10.9 | 22.6 |
| Men | 2491 (41.1) | 69.3 | 7.8 | 22.9 |
| Mean age, years (SD) | | 69.3 (8.9) | 68.6 (8.8) | 68.2 (8.7) |

| | | | | |
|---|---|---|---|---|
| Values are percentages unless otherwise indicated. NA = not applicable, the GWA screen in the Rotterdam study was performed in women only. | | | | |

**Table 2. Haplotype block association with human iris color in region 25.86-26.19 Mb of chromosome 15 from two independent GWA studies from The Netherlands.**

| Sample set | Haplotype Block⁻ | Haplotype | Frequency (%) All | Blue | Inis color frequency (%) Intermediate | Brown | Chi-square | P-values |
|---|---|---|---|---|---|---|---|---|
| ERF study N=192 | 1* | GATA | 70.1 | 75.0 | 74.2 | 65.1 | 3.73 | 0.44 |
| | | GGCG | 23.4 | 17.3 | 19.4 | 28.9 | | |
| | | AGCG | 6.1 | 7.7 | 6.4 | 4.8 | | |
| | 2* | CGA | 69.5 | 72.1 | 72.4 | 49.2 | 14.3 | 0.01 |
| | | CGG | 22.2 | 20.2 | 19.5 | 24.9 | | |
| | | TAG | 15.9 | 7.6 | 8.1 | 26.7 | | |
| | 3** | CTCA | 82.5 | 96.8 | 91.9 | 65.7 | 29.6 | 5.93x10⁻⁵ |
| | | CTTA | 11.2 | | 8.1 | 20.5 | | |
| | | ACTG | 6.1 | 1.3 | 0 | 13.2 | | |
| Rotterdam study N=481 | 1* | GATA | 77.7 | 81.9 | 75.5 | 68.2 | 13.7 | 8.27x10⁻³ |
| | | GGCG | 16.0 | 11.8 | 17.0 | 26.6 | | |
| | | AGCG | 6.0 | 6.2 | 5.3 | 5.1 | | |
| | 2* | CGA | 75.6 | 79.5 | 78.6 | 64.6 | 13.9 | 7.61x10⁻³ |
| | | CGG | 13.4 | 12.9 | 9.7 | 15.3 | | |
| | | TAG | 10.3 | 7.4 | 9.5 | 18.9 | | |
| | 3** | CTCGA | 85.8 | 97.0 | 76.5 | 58.7 | 95.6 | 8.39x10⁻²⁰ |
| | | CTTGA | 7.4 | 1.6 | 14.9 | 19.8 | | |
| | | ACTGG | 5.1 | 1.0 | 6.4 | 16.1 | | |

| | | | | | | | | |
|---|---|---|---|---|---|---|---|---|
| ⁺block 1 includes: rs17567007, rs1800411, rs1448488, rs12910433, block 2 includes: rs16950821, rs8024968, rs6497254, block 3 includes: rs8041209, rs8028689, rs916977, rs4932620, rs2346050; *located within the *OCA2* gene, **located within the *HERC2* gene | | | | | | | | |

**Table 3. Association of four SNPs from the OCA2-HERC2 region with human iris color in the enlarged ERF and Rotterdam study populations (N=8272) together with genotype frequencies in the HapMap populations of Europe, East Aria and Africa.**

| | ERF study (N=2217) | | | | | | Rotterdam study (N=6056) | | | | | | | | Hi HapMap study East Asians+ (N=90) Freq. (%) | | |
|---|---|---|---|---|---|---|---|---|---|---|---|---|---|---|---|---|---|
| | | Iris color frequency (%) | | | | | Iris color frequency (%) | | | | | European (N=60) | | | | | Africans (N=60) |
| SNP | Genotype | N | Blue | Intermediate | Brown | P-value | N | Blue | Intermediate | Brown | P-values | N Freq. (%) N | | | | N | Freq. (%) |
| rs11855019* | TT | 1440 | 54.5 | 22.9 | 22.6 | 4.4×10⁻¹⁰⁰ | 4580 | 77.3 | 8.7 | 14.0 | 5.4×10⁻²¹¹ | 49 | 81.7 | 5 | 5.5 | 3 | 5.0 |
| | TC | 655 | 15.1 | 18.8 | 66.1 | | 1250 | 36.8 | 13.3 | 49.9 | | 11 | 18.3 | 32 | 35.6 | 26 | 43.3 |
| | CC | 64 | 6.3 | 6.2 | 87.5 | | 97 | 15.5 | 9.2 | 75.3 | | 0 | 0 | 53 | 58.9 | 31 | 51.7 |
| rs6497268* | GG | 144 | 153.6 | 21.2 | 25.2 | 5.0×10⁻⁷⁵ | 4233 | 80.3 | 7.7 | 12.0 | 2.8×10⁻²⁶⁷ | 45 | 75.0 | 2 | 2.2 | 8 | 13.3 |
| | GT | 652 | 17.2 | 20.4 | 62.4 | | 1486 | 36.2 | 15.0 | 48.8 | | 15 | 25.0 | 24 | 26.7 | 30 | 50.0 |
| | TT | 67 | 7.5 | 14.9 | 77.6 | | 151 | 17.9 | 11.2 | 70.9 | | 0 | 0 | 64 | 71.1 | 22 | 36.7 |
| rs7495174* | TT | 174 | 949.7 | 21.2 | 29.1 | 4.3×10⁻⁷⁹ | 5273 | 74.6 | 8.9 | 16.5 | 1.4×10⁻²³⁹ | 54 | 90.0 | 7 | 7.8 | 43 | 71.7 |
| | TC | 382 | 3.4 | 20.2 | 76.4 | | 659 | 16.2 | 15.2 | 68.6 | | 6 | 10.0 | 38 | 42.2 | 16 | 26.7 |
| | CC | 17 | 5.9 | 0 | 94.1 | | 34 | 2.9 | 5.9 | 91.2 | | 0 | 0 | 45 | 50.0 | 1 | 1.6 |
| rs916977** | CC | 1543 | 54.8 | 21.3 | 23.9 | 1.9×10⁻¹¹³ | 4572 | 82.4 | 7.3 | 10.3 | <1.0×10⁻³⁰⁰ | 44 | 73.3 | 2 | 2.2 | 0 | 0 |
| | CT | 573 | 6.4 | 22.1 | 69.5 | | 1229 | 18.3 | 18.4 | 63.3 | | 16 | 26.7 | 28 | 31.1 | 6 | 10.0 |
| | TT | 60 | 3.3 | 3.3 | 93.4 | | 105 | 6.6 | 8.6 | 84.8 | | 0 | 0 | 60 | 66.7 | 54 | 90.0 |

| | | | | | | | | | | | | | | | | | |
|---|---|---|---|---|---|---|---|---|---|---|---|---|---|---|---|---|---|
| * located in intron 1 of the *OCA2* gene, ** located in intron 11 of the *HERC2* gene | | | | | | | | | | | | | | | | | |

**Table 4. Haplotype-association of four SNPs from the OCA2-HERC2 region with human iris color in the enlarged ERF and Rotterdam study populations (N=8273) together with haplotype frequencies in the HapMap populations of Europe, East Asia and Africa.**

| | ERF study (N=2217) | | | | Rotterdam study (N=6056) | | | | HapMap (N=30) | | |
|---|---|---|---|---|---|---|---|---|---|---|---|
| | Iris color frequency (%) | | | | Iris color frequency (%) | | | | | Frequency (%) in East Asians | |
| Haplotype | All | Blue | Intermediate | Brown | All | Blue | Intermediate | Brown | Europeans | | Africans |
| TGTC | 75.5 | 92.0 | 79.4 | 55.6 | 81.2 | 91.2 | 72.1 | 55.3 | 82.5 | 11.6 | 5.0 |
| CTCT | 5.5 | 0.6 | 6.0 | 10.5 | 4.5 | 0.9 | 6.7 | 14.5 | 4.2 | 65.8 | 13.3 |
| TTTT | 4.6 | 1.6 | 5.2 | 7.6 | 4.3 | 1.5 | 8.7 | 10.5 | 5.8 | 10.1 | 18.0 |
| CTCC | 4.0 | 0.2 | 2.7 | 9.0 | 0.3 | 0.3 | 1.6 | 3.5 | 0 | 3.4 | 0 |
| TGCT | 0.1 | 0 | 0 | 0.3 | 0.3 | 0 | 0.5 | 1.1 | 0.8 | 1.2 | 1.7 |
| TGTT | 1.3 | 0.3 | 0.7 | 2.7 | 0.9 | 0.3 | 1.5 | 2.6 | 0 | 0.4 | 2.0 |
| CGTT | 3.9 | 0.3 | 2.2 | 8.7 | 1.7 | 0.3 | 2.7 | 5.5 | 2.5 | 1.7 | 29.7 |
| CGTC | 1.0 | 0.5 | 0.6 | 1.7 | 0.8 | 0.7 | 0.8 | 0.8 | 1.7 | 0 | 0 |
| TTTC | 0.3 | 0.1 | 0.3 | 0.4 | 1.0 | 0.8 | 0.8 | 1.9 | 1.7 | 0 | 0 |
| CTTC | 3.3 | 4.2 | 2.8 | 2.5 | 3.5 | 3.9 | 3.2 | 2.6 | 0.8 | 2.7 | 0 |
| CTTT | 0.5 | 0.1 | 0.3 | 1.0 | 0.6 | 0.1 | 1.4 | 1.7 | 0 | 2.4 | 30.3 |
| CGCT | 0 | 0 | 0 | 0 | 0 | 0 | 0 | 0 | 0 | 0.7 | 0 |

| | | | | | | | | | | | |
|---|---|---|---|---|---|---|---|---|---|---|---|
| Haplobpas comist of SNPs in the following order: rs11855019, rs6497268, rs7495174 - all in the *OCA2* gene, and rs916977 in the *HERC2* gene. All haplotype observed in either of the populations are shown,** combines Japanese and Chinese data. | | | | | | | | | | | |

**Table 5. All SNPs associated with iris color on the genome-wide significance level from the Illumina 300 Duo dataset in 733 ERF participants**

| SNP Marker | Gene | P-value genom e-wide | P-value nominal | Genotype | | Iris color frequency | |
|---|---|---|---|---|---|---|---|
| | | | | | | | |
| | | | | | Blue | Intermed. | Brown |
| rs8039195 | HERC2 | <0.01 | <10-16 | TT | 0.5479 | 0.2035 | 0.2485 |
| | | | | TC | 0.0855 | 0.2303 | 0.6842 |
| | | | | CC | 0 | 0 | 1 |
| rs 1667394 | HERC2 | <0.01 | <10-16 | TT | 0.58 | 0.2058 | 0.2141 |
| | | | | TC | 0.0791 | 0.226 | 0.6949 |
| | | | | CC | 0 | 0 | 1 |
| rs7183877 | HERC2 | <0.01 | 1.73E-13 | TT | 0.4863 | 0.2072 | 0.3065 |
| | | | | GT | 0.1098 | 0.2195 | 0.6707 |
| | | | | GG | 0 | 0 | 1 |
| rs16950979 | HERC2 | <0.01 | 3.35E-12 | TT | 0.4824 | 0.2044 | 0.3132 |
| | | | | TC | 0.058 | 0.2319 | 0.7101 |
| | | | | CC | 0 | 0 | 1 |
| rs8028689 | HERC2 | <0.01 | 3.67E-12 | TT | 0.4817 | 0.205 | 0.3133 |
| | | | | TC | 0.058 | 0.2319 | 0.7101 |
| | | | | CC | 0 | 0 | 1 |
| rs2240204 | HERC2 | <0.01 | 3.67E-12 | TT | 0 | 0 | 1 |
| | | | | CT | 0.058 | 0.2319 | 0.7101 |
| | | | | CC | 0.4817 | 0.205 | 0.3133 |
| rs16950987 | HERC2 | <0.01 | 4.39E-12 | TT | 0 | 0 | 1 |
| | | | | CT | 0.058 | 0.2319 | 0.7101 |
| | | | | CC | 0.4799 | 0.2057 | 0.3144 |
| rs1597196 | OCA2 | <0.01 | <10-16 | TT | 0.122 | 0.2195 | 0.6585 |
| | | | | GT | 0.25 | 0.2069 | 0.5431 |
| | | | | GG | 0.5779 | 0.206 | 0.2161 |
| rs4778138 | OCA2 | <0.01 | <10-16 | TT | 0.5756 | 0.2144 | 0.2099 |
| | | | | TC | 0.1779 | 0.2115 | 0.6106 |
| | | | | CC | 0.05 | 0 | 0.95 |
| rs4778241 | OCA2 | <0.01 | <10-16 | TT | 0.5578 | 0.2133 | 0.2289 |
| | | | | GT | 0.1961 | 0.201 | 0.6029 |
| | | | | GG | 0.1176 | 0.1176 | 0.7647 |
| rs7495174 | OCA2 | <0.01 | <10-16 | TT | 0.5226 | 0.2134 | 0.264 |
| | | | | TC | 0.0357 | 0.1875 | 0.7768 |
| | | | | CC | 0 | 0 | 1 |
| rs4778232 | OCA2 | <0.01 | 5.00E-14 | TT | 0.1636 | 0.1818 | 0.6545 |
| | | | | CT | 0.3404 | 0.1879 | 0.4716 |
| | | | | CC | 0.5629 | 0.2275 | 0.2096 |
| rs7179994 | OCA2 | <0.01 | 5.46E-14 | TT | 0.5315 | 0.2017 | 0.2668 |
| | | | | TC | 0.2034 | 0.226 | 0.5706 |
| | | | | CC | 0.1765 | 0.1765 | 0.6471 |
| rs3794604 | OCA2 | <0.01 | 1.99E-12 | TT | 0.125 | 0.1667 | 0.7083 |
| | | | | CT | 0.2556 | 0.2056 | 0.5389 |
| | | | | CC | 0.5226 | 0.2108 | 0.2667 |
| rs1635168 | HERC2 | <0.01 | 2.74E-13 | TT | 0 | 0 | 1 |
| | | | GT | GT | 0.0548 | 0.2329 | 0.7123 |
| | | | | GG | 0.484 | 0.205 | 0.3109 |
| rs8024968 | OCA2 | <0.01 | 4.12E-12 | TT | 0.125 | 0.1667 | 0.7083 |
| | | | | CT | 0.2623 | 0.2022 | 0.5355 |
| | | | | CC | 0.5216 | 0.2112 | 0.2672 |
| rs2594935 | OCA2 | <0.01 | 1.00E-11 | TT | 0.5737 | 0.2038 | 0.2226 |
| | | | | CT | 0.33 | 0.1987 | 0.4714 |
| | | | | CC | 0.2182 | 0.2727 | 0.5091 |
| rs728405 | OCA2 | <0.01 | 1.95E-10 | TT | 0.5314 | 0.1816 | 0.287 |
| | | | | TG | 0.2624 | 0.2525 | 0.4851 |
| | | | | GG | 0.1304 | 0.3043 | 0.5652 |

### References

Abecasis GR, Cherny SS, Cookson WO, Cardon LR (2002) Merlin-rapid analysis of dense genetic maps using sparse gene flow trees. Nat Genet 30:97-101
Almasy L, Blangero J (1998) Multipoint quantitative-trait linkage analysis in general pedigrees. Am J Hum Genet 62:1198-1211
Aulchenko YS, Heutink P, Mackay I, Bertoli-Avella AM, Pullen J, Vaessen N, Rademaker TA, Sandkuijl LA, Cardon L, Oostra B, van Duijn CM (2004) Linkage disequilibrium in young genetically isolated Dutch population. Eur J Hum Genet 12:527-534
Aulchenko YS, Ripke S, Isaacs A, van Duijn CM (2007) GenABEL: an R library for genome-wide association analysis. Bioinformatics 23:1294-1296
Beals RL, Hoijer H (1965) An introduction to anthropology. Allyn and Bacon, Boston, MA. Bito LZ, Matheny A, Cruickshanks KJ, Nondahl DM, Carino OB (1997) Eye color changes past early childhood. The Louisville Twin Study. Arch Ophthalmol 115:659-663
Brilliant MH (2001) The mouse p (pink-eyed dilution) and human P genes, oculocutaneous albinism type 2 (OCA2), and melanosomal pH. Pigment Cell Res 14:86-93
Butler MG (1989) Hypopigmentation: a common feature of Prader-Labhart-Willi syndrome. Am J Hum Genet 45:140-146
Cavalli-Sforza LL, Menozzi P, Piazza A (1994) The history and geography of human genes. Princeton University Press, Princeton
Chai JH, Locke DP, Greally JM, Knoll JH, Ohta T, Dunai J, Yavor A, Eichler EE, Nicholls RD (2003) Identification of four highly conserved genes between breakpoint hotspots BP1 and BP2 of the Prader-Willi/Angelman syndromes deletion region that have undergone evolutionary transposition mediated by flanking duplicons. Am J Hum Genet 73:898-925
Davenport GC, Davenport CB (1907) Heredity of eye-color in man. Science 26:590-592
Devlin B, Roeder K (1999) Genomic control for association studies. Biometrics 55:997-1004
Duffy DL, Box NF, Chen W, Palmer JS, Montgomery GW, James MR, Hayward NK, Martin NG, Sturm RA (2004) Interactive effects of MC1R and OCA2 on melanoma risk phenotypes. Hum Mol Genet 13:447-461
Duffy DL, Montgomery GW, Chen W, Zhao ZZ, Le L, James MR, Hayward NK, Martin NG, Sturm RA (2007) A three-single-nucleotide polymorphism haplotype in intron 1 of OCA2 explains most human eye-color variation. Am J Hum Genet 80:241-252
Eiberg H, Mohr J (1987) Major genes of eye color and hair color linked to LU and SE. Clin Genet 31:186-191
Eiberg H, Mohr J (1996) Assignment of genes coding for brown eye colour (BEY2) and brown hair colour (HCL3) on chromosome 15q. Eur J Hum Genet 4:237-241
Frost P (2006) European hair and eye color. A case of frequency-dependent sexual selection? Evolution and Human Behavior 27:85-103
Frudakis T, Terravainen T, Thomas M (2007) Multilocus OCA2 genotypes specify human iris color. Hum Genet Online published ahead of print:DOI: 10.1007/s00439-00007-00401-00438
Frudakis T, Thomas M, Gaskin Z, Venkateswarlu K, Chandra KS, Ginjupalli S, Gunturi S, Natrajan S, Ponnuswamy VK, Ponnuswamy KN (2003) Sequences associated with human iris pigmentation. Genetics 165:2071-2083
Gabriel SB, Schaffner SF, Nguyen H, Moore JM, Roy J, Blumenstiel B, Higgins J, DeFelice M, Lochner A, Faggart M, Liu-Cordero SN, Rotimi C, Adeyemo A, Cooper R, Ward R, Lander ES, Daly MJ, Altshuler D (2002) The structure of haplotype blocks in the human genome. Science 296:2225-2229
Graf J, Hodgson R, van Daal A (2005) Single nucleotide polymorphisms in the MATP gene are associated with normal human pigmentation variation. Hum Mutat 25:278-284
Hanley JA, McNeil BJ (1982) The meaning and use of the area under a receiver operating characteristic (ROC) curve. Radiology 143:29-36
Hofman A, Grobbee DE, de Jong PT, van den Ouweland FA (1991) Determinants of disease and disability in the elderly: the Rotterdam Elderly Study. Eur J Epidemiol 7:403-422
Imesch PD, Bindley CD, Khademian Z, Ladd B, Gangnon R, Albert DM, Wallow IH (1996) Melanocytes and iris color. Electron microscopic findings. Arch Ophthalmol 114:443-447
Imesch PD, Wallow IH, Albert DM (1997) The color of the human eye: a review of morphologic correlates and of some conditions that affect iridial pigmentation. Surv Ophthalmol 41 Suppl 2:S117-123
Jannot AS, Meziani R, Bertrand G, Gerard B, Descamps V, Archimbaud A, Picard C, Ollivaud L, Basset-Seguin N, Kerob D, Lanternier G, Lebbe C, Saiag P, Crickx B, Clerget-Darpoux F, Grandchamp B, Soufir N, Melan C (2005) Allele variations in the OCA2 gene (pink-eyed-dilution locus) are associated with genetic susceptibility to melanoma. Eur J Hum Genet 13:913-920
Ji Y, Rebert NA, Joslin JM, Higgins MJ, Schultz RA, Nicholls RD (2000) Structure of the highly conserved HERC2 gene and of multiple partially duplicated paralogs in human. Genome Res 10:319-329
Ji Y, Walkowicz MJ, Buiting K, Johnson DK, Tarvin RE, Rinchik EM, Horsthemke B, Stubbs L, Nicholls RD (1999) The ancestral gene for transcribed, low-copy repeats in the Prader-Willi/Angelman region encodes a large protein implicated in protein trafficking, which is deficient in mice with neuromuscular and spermiogenic abnormalities. Hum Mol Genet 8:533-542
Kanetsky PA, Swoyer J, Panossian S, Holmes R, Guerry D, Rebbeck TR (2002) A polymorphism in the agouti signaling protein gene is associated with human pigmentation. Am J Hum Genet 70:770-775
King RA, Wiesner GL, Townsend D, White JG (1993) Hypopigmentation in Angelman syndrome. Am J Med Genet 46:40-44
Lettice LA, Heaney SJ, Purdie LA, Li L, de Beer P, Oostra BA, Goode D, Elgar G, Hill RE, de Graaff E (2003) A long-range Shh enhancer regulates expression in the developing limb and fin and is associated with preaxial polydactyly. Hum Mol Genet 12:1725-1735
Miller SA, Dykes DD, Polesky HF (1988) A simple salting out procedure for extracting DNA from human nucleated cells. Nucleic Acids Res 16:1215
Pardo LM, MacKay I, Oostra B, van Duijn CM, Aulchenko YS (2005) The effect of genetic drift in a young genetically isolated population. Ann Hum Genet 69:288-295
Posthuma D, Visscher PM, Willemsen G, Zhu G, Martin NG, Slagboom PE, de Geus EJ, Boomsma DI (2006) Replicated linkage for eye color on 15q using comparative ratings of sibling pairs. Behav Genet 36:12-17
Purcell S, Neale B, Todd-Brown K, Thomas L, Ferreira MAR, Bender D, Maller J, de Bakker PIW, Daly MJ, Sham PC (2007) PLINK: a toolset for whole-genome association and population-based linkage analyses. Am J Hum Genet:in press (preprint online published May 2007)
Ramsay M, Colman MA, Stevens G, Zwane E, Kromberg J, Farrall M, Jenkins T (1992) The tyrosinase-positive oculocutaneous albinism locus maps to chromosome 15q11.2-q12. Am J Hum Genet 51:879-884
Rebbeck TR, Kanetsky PA, Walker AH, Holmes R, Halpern AC, Schuchter LM, Elder DE, Guerry D (2002) P gene as an inherited biomarker of human eye color. Cancer Epidemiol Biomarkers Prev 11:782-784
Rinchik EM, Bultman SJ, Horsthemke B, Lee ST, Strunk KM, Spritz RA, Avidano KM, Jong MT, Nicholls RD (1993) A gene for the mouse pink-eyed dilution locus and for human type II oculocutaneous albinism. Nature 361:72-76
Schaid DJ, Rowland CM, Tines DE, Jacobson RM, Poland GA (2002) Score tests for association between traits and haplotypes when linkage phase is ambiguous. Am J Hum Genet 70:425-434
Service S, DeYoung J, Karayiorgou M, Roos JL, Pretorious H, Bedoya G, Ospina J, et al. (2006) Magnitude and distribution of linkage disequilibrium in population isolates and implications for genome-wide association studies. Nat Genet 38:556-560
Sokal RR, Oden NL (1978) Spatial autocorrelation in biology 1. Metodology. Biological Journal of the Linnean Society 10:199-228
Spritz RA, Fukai K, Holmes SA, Luande J (1995) Frequent intragenic deletion of the P gene in Tanzanian patients with type II oculocutaneous albinism (OCA2). Am J Hum Genet 56:1320-1323
Staehling-Hampton K, Proll S, Paeper BW, Zhao L, Charmley P, Brown A, Gardner JC, Galas D, Schatzman RC, Beighton P, Papapoulos S, Hamersma H, Brunkow ME (2002) A 52-kb deletion in the SOST-MEOX1 intergenic region on 17q12-q21 is associated with van Buchem disease in the Dutch population. Am J Med Genet 110:144-152
Steinthorsdottir V, Thorleifsson G, Reynisdottir I, Benediktsson R, Jonsdottir T, Walters GB, Styrkarsdottir U, et al. (2007) A variant in CDKAL1 influences insulin response and risk of type 2 diabetes. Nat Genet 39:770-775
Sturm RA, Frudakis TN (2004) Eye colour: portals into pigmentation genes and ancestry. Trends Genet 20:327-332
Wielgus AR, Sarna T (2005) Melanin in human irides of different color and age of donors. Pigment Cell Res 18:454-464
Wiesner GL, Bendel CM, Olds DP, White JG, Arthur DC, Ball DW, King RA (1987) Hypopigmentation in the Prader-Willi syndrome. Am J Hum Genet 40:431-442
Zhu G, Evans DM, Duffy DL, Montgomery GW, Medland SE, Gillespie NA, Ewen KR, Jewell M, Liew YW, Hayward NK, Sturm RA, Trent JM, Martin NG (2004) A genome scan for eye color in 502 twin families: most variation is due to a QTL on chromosome 15q. Twin Res 7:197-210

## Claims

1. A method to predict iris color of a human from a nucleic acid sample comprising assaying for one or more polymorphisms in the *HERC2* gene and on basis of the results from the assay predicting the eye color,
wherein the one or more polymorphisms are selected from the group consisting of rs916977, rs8028689, rs6497287, rs8041209, rs6497292, rs2240202, rs2346050, rs12592730, rs7183877, rs2240204, rs8039195, rs16950979, rs16950987, rs1667394, and rs1635168 and any marker in the *HERC2* gene in genetic linkage disequilibrium with said polymorphisms.

2. A method according to claim 1, wherein the polymorphism is rs916977.

3. A method according to claim 2, wherein the prediction is blue eyes when the rs916977 polymorphism has the sequence CC considering both homologue chromosomes (or GG when considering the complementary bases), brown eyes when the nucleotide sequence is TT (or AA when considering the complementary bases).

4. A method according to claim 1 where the polymorphisms predict the eye colors in the following matter: rs8028689: CC brown and TT blue, rs6497287: AA blue and GG brown, rs8041209: AA brown and CC blue, rs6497292: AA blue and GG brown, rs2240202: AA brown and GG blue, rs2346050: AA blue and GG brown, rs12592730: AA brown and GG blue, rs7183877: TT blue and GG brown, rs2240204: TT brown and CC blue, rs8039195: TT blue and CC brown, rs16950979: AA blue and CC brown, rs16950987: TT brown and CC blue, rs1667394: TT blue and CC brown, and rs1635168: TT brown and GG blue.

5. A method of indicating human iris color by detecting a nucleotide occurrence for a single nucleotide polymorphism (SNP), comprising:
(i) incubating a sample comprising a polynucleotide with a specific binding pair member, wherein the specific binding pair member specifically binds at or near a polynucleotide suspected of being polymorphic, wherein the polynucleotide comprises one of the nucleotide occurrences corresponding to at least one of the polymorphisms rs916977, rs8028689, rs6497287, rs8041209, rs6497292, rs2240202, rs2346050, rs12592730, rs7183877, rs2240204, rs8039195, rs16950979, rs16950987, rs1667394, and rs1635168 or any combination thereof or any marker in the *HERC2* gene in genetic linkage, disequilibrium with said polymorphisms; and ii) detecting selective binding of the specific binding pair member, wherein selective binding is indicative of the presence of the nucleotide occurrence, thereby detecting the nucleotide occurrence for the polymorphism.

## Patentansprüche

1. Verfahren zur Vorhersage der Irisfarbe bei einem Menschen anhand einer Nukleinsäureprobe, umfassend die Untersuchung auf einen oder mehrere Polymorphismen im *HERC2*-Gen und Vorhersage der Augenfarbe auf der Grundlage der Untersuchungsergebnisse, wobei der eine oder die mehreren Polymorphismen ausgewählt sind aus der Gruppe, bestehend aus rs916977, rs8028689, rs6497287, rs8041209, rs6497292, rs2240202, rs2346050, rs12592730, rs7183877, rs2240204, rs8039195, rs16950979, rs16950987, rs1667394 und rs1635168 und jeder Markierung im *HERC2*-Gen in genetischem Kopplungsungleichgewicht mit den Polymorphismen.

2. Verfahren nach Anspruch 1, wobei der Polymorphismus rs916977. ist.

3. Verfahren nach Anspruch 2, wobei die Vorhersage blaue Augen ist, wenn der rs916977 Polymorphismus unter Berücksichtigung beider homologer Chromosomen die Sequenz CC hat (oder GG, unter Berücksichtigung der komplementären Basen), oder braune Augen, wenn die Nukleotidsequenz TT ist (oder AA, unter Berücksichtigung der komplementären Basen).

4. Verfahren nach Anspruch 1, wobei die Polymorphismen die Augenfarbe wie folgt vorhersagen: rs8028689: CC braun und TT blau, rs6497287: AA blau und GG braun, rs8041209: AA braun und CC blau, rs6497292: AA blau und GG braun, rs2240202: AA braun und GG blau, rs2346050: AA blau und GG braun, rs12592730: AA braun und GG blau, rs7183877: TT blau und GG braun, rs2240204: TT braun und CC blau, rs8039195: TT blau und CC braun, rs16950979: AA blau und CC braun, rs16950987: TT braun und CC blau, rs1667394: TT blau und CC braun, und rs1635168: TT braun und GG blau.

5. Verfahren zur Anzeige der menschlichen Irisfarbe durch Detektieren eines Nukleotidauftretens für einen einzelnen Nukleotid-Polymorphismus (SNP), umfassend:
(i) Inkubieren einer Probe, umfassend ein Polynukleotid mit einem spezifischen bindenden Nukleotid-Paarelement, wobei das spezifische bindende Paarelement sich spezifisch an oder nahe einem Polynukleotid bindet, von dem vermutet wird, dass es polymorph ist, wobei das Polynukleotid eines der Nukleotidauftreten entsprechend mindestens einem der Polymorphismen rs916977, rs8028689, rs6497287, rs8041209, rs6497292, rs2240202, rs2346050, rs12592730, rs7183877, rs2240204, rs8039195, rs16950979, rs16950987, rs1667394 und rs1635168 oder Kombinationen davon oder eine beliebige Markierung im *HERC2*-Gen in genetischem Kopplungsungleichgewicht mit den Polymorphismen umfasst, und (ii) Detektieren der selektiven Bindung des spezifisch bindenden Paarelements, wobei selektive Bindung ein Hinweis auf die Anwesenheit des Nukleotidauftretens ist, wodurch das Nukleotidauftreten für den Polymorphismus detektiert wird.

## Revendications

1. Procédé pour prédire la couleur de l'iris d'un humain d'après un échantillon d'acide nucléique comprenant le test d'un ou plusieurs polymorphismes dans le gène *HERC2* et sur la base des résultats du test la prédiction de la couleur des yeux, où le un ou plusieurs polymorphismes sont choisis dans le groupe consistant en rs916977, rs8028689, rs6497287, rs8041209, rs6497292, rs2240202, rs23446050, rs12592730, rs7183877, rs2240204, rs8039195, rs16950979, rs16950987, rs1667394 et rs1635168 et tout marqueur dans le gène *HERC2* en déséquilibre de liaison génétique avec lesdits polymorphismes.

2. Procédé selon la revendication 1, où le polymorphisme est rs916977.

3. Procédé selon la revendication 2, où la prédiction est des yeux bleus quand le polymorphisme rs916977 a la séquence CC en considérant les deux chromosomes homologues (ou GG quand on considère les bases complémentaires), des yeux bruns quand la séquence nucléotidique est TT (ou AA quand on considère les bases complémentaires).

4. Procédé selon la revendication 1 où les polymorphismes prédisent les couleurs des yeux de la manière suivante : rs8028689 : CC brun et TT bleu, rs6497287 : AA bleu et GG brun, rs8041209 : AA brun et CC bleu, rs6497292 : AA bleu et GG brun, rs2230202 : AA brun et GG bleu, rs22346050 : AA bleu et GG brun, rs12592730 : AA brun et GG bleu, rs7183877 : TT bleu et GG brun, rs2240204 : TT brun et CC bleu, rs8039195 : TT bleu et CC brun, rs16950979 : AA bleu et CC brun, rs16950987 : TT brun et CC bleu, rs1667394 : TT bleu et CC brun, et rs1635168 : TT brun et GG bleu.

5. Procédé d'indication de la couleur de l'iris humain par détection d'une occurrence de nucléotide pour un polymorphisme d'un seul nucléotide (SNP) comprenant :
(i) incubation d'un échantillon comprenant un polynucléotide avec un membre d'une paire à liaison spécifique, où le membre d'une paire à liaison spécifique se lie spécifiquement à ou à proximité d'un polynucléotide suspecté d'être polymorphe, où le polynucléotide comprend l'une des occurrences de nucléotide correspondant en au moins l'un des polymorphismes rs916977, rs8028689, rs6497287, rs8041209, rs6497292, rs2240202, rs23446050, rs12592730, rs7183877, rs2240204, rs8039195, rs16950979, rs16950987, rs1667394 et rs1635168 ou toute combinaison de ceux-ci ou tout marqueur dans le gène *HERC2* en déséquilibre de liaison génétique avec lesdits polymorphismes, et (ii) la détection d'une liaison sélective du membre d'une paire à liaison spécifique, où la liaison sélective indique la présence de l'occurrence de nucléotide, en détectant ainsi l'occurrence de nucléotide pour le polymorphisme.
